(19) Europäisches Patentamt
European Patent Office
Office européen des brevets

(11) **EP 4 454 545 A1**

(12) **EUROPEAN PATENT APPLICATION**

(43) Date of publication:
**30.10.2024 Bulletin 2024/44**

(21) Application number: **24170970.8**

(22) Date of filing: **18.04.2024**

(51) International Patent Classification (IPC):
*A61B 5/021* (2006.01)   *A61B 5/024* (2006.01)
*A61B 5/00* (2006.01)   *G06F 3/00* (2006.01)

(52) Cooperative Patent Classification (CPC):
**A61B 5/02108; A61B 5/02427; A61B 5/6897;
A61B 5/6898; A61B 5/7221; A61B 5/743;
G06F 3/00;** A61B 5/02007; A61B 5/02028;
A61B 5/02405; A61B 5/0816; A61B 5/7264;
A61B 2560/0462; A61B 2562/046

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.04.2023 KR 20230056139**

(71) Applicant: **Samsung Display Co., Ltd.
Yongin-si, Gyeonggi-do 17113 (KR)**

(72) Inventor: **Kim, Chul
Yongin-si, Gyeonggi-do (KR)**

(74) Representative: **Gulde & Partner
Patent- und Rechtsanwaltskanzlei mbB
Wallstraße 58/59
10179 Berlin (DE)**

(54) **DISPLAY DEVICE**

(57)    A display device including: display pixels arranged in a display area of a display panel; light sensing pixels arranged in the display area; a display scan driver configured to drive the display pixels to emit light; a light sensing scan driver configured to drive the light sensing pixels to sense light; and a main driving circuit configured to measure a user's biometric information using light sensing signals received from the light sensing pixels, wherein the main driving circuit displays a preset user behavior guidance program screen during a measurement period of biometric information to guide a user's biometric signal detection process, and displays user behavior guidance information and the biometric information measurement result using the biometric signal in real time.

**FIG. 8**

**Description**

1. Technical Field

The present disclosure relates to a display device capable of measuring biometric information, including but not limited to blood pressure, and heart rate.

2. Description of the Related Art

As society becomes increasingly information-oriented, the demand for display devices continues to rise. Display devices are now integral to various electronic devices including, but not limited to, smartphones digital cameras, laptops, tablets, navigation systems, and smart televisions. For portable display devices, such as smartphones, and tablets, various functions are offered including, but not limited to, image capturing, fingerprint recognition, and face recognition.

Recently, with the increasing focus on the healthcare industry, there have been developments in methods to more easily obtain health-related biometric information. For example, there have been efforts to substitute a traditional blood pressure measuring device that uses an oscillometric method with a portable blood pressure measuring device. However, such portable blood pressure measuring devices need their own separate light source, sensor, and display. This means, individuals need to carry the portable blood pressure measuring device separately, in addition to their smartphones or tablets, causing a degree of inconvenience.

Recently, efforts have been made to combine a portable display device like a smartphone and a tablet with a portable blood pressure measuring device. Further, there is a need for methods that allow the measurement of various pieces of biometric information such as heart rate, heart rate variability, respiration, cardiovascular disease, oxygen saturation, and more, using portable display devices in addition to measuring blood pressure.

**SUMMARY**

Embodiments of the present disclosure provide a display device capable of measuring biometric information by selecting the most accurate pulse wave signals from among a plurality of pulse wave signals detected by a plurality of display devices or a pulse wave measuring module.

Embodiments of the present disclosure also provide a display device that measures various biometric information such as blood pressure, heart rate, heart rate variability, respiratory rate, and oxygen saturation, and feeds back this biometric information to an application screen in real time during a user behavior guidance information display period for mental and physical stabilization.

According to an embodiment of the present disclosure, there is provided a display device including: display pixels arranged in a display area of a display panel; light sensing pixels arranged in the display area; a display scan driver configured to drive the display pixels to emit light; a light sensing scan driver configured to drive the light sensing pixels to sense light; and a main driving circuit configured to measure a user's biometric information using light sensing signals received from the light sensing pixels, wherein the main driving circuit displays a preset user behavior guidance program screen during a measurement period of biometric information to guide a user's biometric signal detection process, and displays user behavior guidance information and the biometric information measurement result using the biometric signal in real time.

According to an embodiment of the present disclosure, there is provided a display device including: display pixels arranged in a display area of a display panel; light sensing pixels arranged in the display area; infrared light emitting pixels arranged in the display area; a display scan driver configured to drive the display pixels to emit light; a light sensing scan driver configured to drive the light sensing pixels to sense light; a touch sensing unit disposed on a front surface of the display panel to sense a user's touch and output a touch sensing signal; a touch driving circuit configured to generate touch data and touch coordinate data according to a magnitude change and an output position of the touch sensing signal; and a main driving circuit configured to measure a user's biometric information using light sensing signals received from the light sensing pixels, wherein the main driving circuit displays a preset user behavior guidance program screen during a biometric information measurement period to guide the user's pulse wave signal detection process, and displays user behavior guidance information and the biometric information measurement result using the biometric signal in real time.

According to an embodiment of the present disclosure, the main driving circuit may detect pulse wave signals among the user's biometric signals by using the light sensing signals during the measurement period of biometric information, analyze a high pulse cycle and a high pulse cycle change of the pulse wave signals, a high pulse magnitude and a magnitude change of the pulse wave signals, a low pulse magnitude and a magnitude change of the pulse wave signals, a high pulse waveform change, a period in which the high pulse reaches its peak, and the difference in magnitude of the pulse wave signals respectively detected by green light and red light, and measure at least one of biometric information among blood pressure, heart rate, heart rate variability, respiratory rate, blood vessel elasticity, a cardiovascular disease analysis result, and oxygen saturation according to each analysis result and displays the biometric information on the user behavior guidance program screen.

According to an embodiment of the present disclosure, the main driving circuit may display meditation-related user behavior guidance information on the user behavior guidance program screen, and a notification screen for displaying the user's heart rate and heart rate variability is displayed when the meditation-related user behavior guidance information is displayed. Hereinafter, the meditation-related user action guidance information may refer to guidance information displayed on the meditation program display screen, or guidance information set to be displayed on the meditation program display screen.

According to an embodiment of the present disclosure, the main driving circuit may detect and count a generation cycle of the high pulses of the pulse wave signals to detect the user's heart rate and heart rate variability in real time, and display the user's heart rate and heart rate variability in real time.

According to an embodiment of the present disclosure, the main driving circuit may display a meditation screen on the user behavior guidance program screen, output sound, and provide feedback for the user's respiratory rate and breathing timing information during a period in which the meditation screen is displayed.

According to an embodiment of the present disclosure, the main driving circuit may sequentially detect a generation cycle and the magnitude of the low pulses of the pulse wave signals,
analyze a cycle in which the magnitude of the low pulses increases and a cycle in which the magnitude of the low pulses decreases to detect the user's breathing timing information and the respiratory rate, and display the user's breathing timing information and the respiratory rate in real time.

According to an embodiment of the present disclosure, the main driving circuit may display a breathing timing guidance screen on a display window for showing breathing timing guidance information including a respiratory rate and inhalation and exhalation periods to a user during the period in which the user behavior guidance program is being executed.

According to an embodiment of the present disclosure, the main driving circuit may compare the user's inhalation and exhalation period detected in real time during the execution period of the user behavior guidance program and respiratory rate to a preset range for the inhalation period and exhalation period for meditation and a range for a standard respiratory rate, respectively, and when the user's inhalation and exhalation period and the respiratory rate may be included within the preset range for the inhalation period and exhalation period for meditation and the range below the standard respiratory rate, breathing timing guidance information including preset ranges for the inhalation period and exhalation period for meditation and the standard respiratory rate is displayed in real time on the display window.

According to an embodiment of the present disclosure, the main driving circuit may display a touch sensing area of the user's body part while the user behavior guidance program screen is displayed on the display area, display waveforms of the pulse wave signals detected in real time on a display window of the user behavior guidance program screen to guide the user's pulse wave signal detection process, and display a detection period of the pulse wave signals and a period during which the pulse wave signals need to be detected to adequately measure the biometric information.

According to an embodiment of the present disclosure, the main driving circuit may generate the pulse wave signals corresponding to magnitudes of the light sensing signals and changes in the magnitudes of the light sensing signals, analyze the high pulse magnitude change and the low pulse magnitude change of the pulse wave signals to calculate an average magnitude value of the high pulses and an average magnitude value of the low pulses in real time, and set a normal pulse wave signal detection period or an inaccurate pulse wave signal detection period in real time according to a comparison result of the average magnitude value of the high pulses to a preset high threshold and the comparison result of the average magnitude value of the low pulses to a preset low threshold.

According to an embodiment of the present disclosure, the main driving circuit may set the inaccurate pulse wave signal detection period when the average magnitude value of the high pulses is smaller than the preset high threshold during a plurality of preset frame periods or when the average magnitude value of the low pulses is smaller than the preset low threshold during the plurality of preset frame periods.

According to an embodiment of the present disclosure, the main driving circuit may generate a capacitance profile or an image based on values of the light sensing signals detected by the light sensing pixels, detect a touch area of the user's body part based on the capacitance profile or the image, and set an incorrect pulse wave signal detection period when the detected touch area is smaller than a reference area.

According to an embodiment of the present disclosure, the main driving circuit may display a first touch guide message on the user behavior guidance program screen so that the user's body part can be accurately touched and a touch state can be maintained during the inaccurate pulse wave signal detection period, and display the waveforms of the pulse wave signals in real time on the display window and the touch guide message indicating a warning.

According to an embodiment of the present disclosure, the main driving circuit may display a second touch guide message on the user behavior guidance program screen so that a normal touch state can be maintained when the normal pulse wave signal detection period is set, and display the waveforms of the pulse wave signals in real time on the display window and the touch guide message indicating a normal condition.

According to an embodiment of the present disclosure, the main driving circuit may generate first pulse wave signals corresponding to magnitudes of the light sensing signals and changes in the magnitudes of the light sensing signals, receive at least one second pulse wave signal or an $n^{th}$ pulse wave signal from a main driving circuit formed in at least one other adjacent display device, compare and analyzes the first and second pulse wave signals or at least two pulse wave signals among the first to $n^{th}$ pulse wave signals, and measures the biometric information by selecting and using any one pulse wave signal according to the analysis result.

According to an embodiment of the present disclosure, the main driving circuit may calculate and compares an average magnitude value of high pulses and an average magnitude value of low pulses of the first and second pulse wave signals or the first to $n^{th}$ pulse wave signals, selects any one of the pulse wave signals according to the comparison result, and measures the biometric information by analyzing the selected pulse wave signal.

According to an embodiment of the present disclosure, the main driving circuit may compare average magnitude values of high pulses of the first and second pulse wave signals or the first to $n^{th}$ pulse wave signals with each other, and selects any one pulse wave signal having the largest average magnitude value of the high pulses to measure the biometric information, measure the biometric information by selecting any one pulse wave signal having a median value among the average magnitude values of the high pulses compared with each other, compare average magnitude values of low pulses of the first and second pulse wave signals or the first to $n^{th}$ pulse wave signals with each other, and selects any one pulse wave signal having the smallest average magnitude value of the low pulses to measure the biometric information , or measure the biometric information by selecting any one pulse wave signal having a median value among the average magnitude values of the low pulses compared with each other.

According to an embodiment of the present disclosure, the main driving circuit may calculate an average magnitude value of high pulses and an average magnitude value of low pulses of the first and second pulse wave signals or the first to $n^{th}$ pulse wave signals, compares the calculated average magnitude value of the high pulses with a preset high threshold, compares the average magnitude value of the low pulses with a preset low threshold, selects any one of the pulse wave signals according to the comparison result, and measures the biometric information by analyzing the selected pulse wave signal.

In accordance with a display device according to embodiments of the present disclosure, when biometric information is measured by selecting more accurately detected pulse wave signals from among a plurality of pulse wave signals detected by a plurality of display devices or a pulse wave measuring module, biometric information detection accuracy and reliability can be improved.

In addition, by providing real-time feedback and reference to heart rate variability, respiratory rate, and breathing timing information through a user behavior guidance program during a meditation period or mental and physical stabilization period, it is possible to assist the user to stabilize themselves mentally and physically and increase the user's health and satisfaction.

## BRIEF DESCRIPTION OF THE DRAWINGS

The above and other features of the present disclosure will become more apparent by describing in detail embodiments thereof with reference to the attached drawings, in which:

FIG. 1 is a perspective view illustrating a display device according to one embodiment;
FIG. 2 is a plan view illustrating an arrangement structure of a display panel and a display driving circuit shown in FIG. 1;
FIG. 3 is a side view showing the configuration of the display device shown in FIG. 1;
FIG. 4 is a side view of another embodiment illustrating the configuration of the display device shown in FIG. 1;
FIG. 5 is a layout diagram schematically showing an example of the display panel illustrated in FIGS. 1 to 4;
FIG. 6 is a layout diagram illustrating a display area according to one embodiment;
FIG. 7 is a circuit diagram illustrating a display pixel and a light sensing pixel according to one embodiment;
FIG. 8 is a diagram showing an image display screen and a body part touch area detection process during a biometric information measurement period;
FIG. 9 is a graph showing a pulse wave signal detected in real time during a biometric information measurement period and an inaccurate pulse wave signal detection process;
FIG. 10 is an image diagram showing a state in which a pulse wave signal is inaccurately detected during a biometric information measurement period;
FIG. 11 is a diagram showing an image display screen displayed when an inaccurate pulse wave signal is detected during a biometric information measurement period;
FIG. 12 is a diagram showing a biometric information measurement result through a mobile display device and a watch type display device;

FIG. 13 is a diagram showing a biometric information measuring method using a mobile display device and an image display screen;

FIG. 14 is a diagram showing a biometric information measuring method using a watch-type display device and an image display screen;

FIG. 15 is a diagram showing a biometric information measuring method using a watch-type display device with a touch sensing area formed on the rear surface of a mobile display device and an image display screen;

FIG. 16 is a diagram showing a biometric information measuring method with a touch sensing area formed on the rear surface of a watch-type display device and an image display screen;

FIG. 17 is a diagram showing a biometric information measuring method using light reflected from a user's face;

FIG. 18 is a diagram showing a biometric information measuring method using a pulse wave measuring module of one embodiment connected to a mobile display device in a wireless communication method;

FIG. 19 is a diagram showing another biometric information measuring method using a pulse wave measuring module of one embodiment connected to a mobile display device in a wireless communication method;

FIG. 20 is a diagram showing a biometric information measuring method using a pulse wave measuring module of another embodiment connected to a mobile display device in a wireless communication method;

FIG. 21 is a diagram showing a biometric information measuring method using a pulse wave measuring module of still another embodiment connected to a mobile display device in a wireless communication method;

FIG. 22 is a diagram showing a biometric information measuring method using a laptop computer;

FIG. 23 is a graph showing a comparison of a mobile display device, a watch-type display device, pulse wave signals detected in the touch sensing area on the rear surface of the mobile display device, and pulse wave signals detected in the touch sensing area on the rear surface of the watch type display device;

FIG. 24 is a flowchart illustrating a process of selecting a pulse wave signal for measuring biometric information by comparing pulse wave signals respectively detected in a mobile display device and a watch type display device;

FIG. 25 is a flowchart illustrating a process of selecting a pulse wave signal for measuring biometric information among pulse wave signals respectively detected in the touch sensing areas on the front surface and the rear surface of a mobile display device;

FIG. 26 is a graph illustrating a method for calculating blood pressure information among biometric information according to one embodiment;

FIG. 27 is a diagram illustrating a blood pressure information calculation method using a machine learning algorithm according to another embodiment of the present disclosure;

FIG. 28 is a graph illustrating a method for calculating information on blood vessel elasticity among biometric information according to one embodiment;

FIG. 29 is a diagram illustrating a heart rate variability change state measured in real time by feeding it back to a mobile and watch-type display device as an application program screen;

FIG. 30 is a diagram illustrating the respiratory rate and breathing timing information measured in real time by feeding them back to a watch-type display device as an application program screen;

FIG. 31 is a diagram illustrating respiratory rate and breathing timing information measured in real time by feeding them back to a mobile display device as an application screen;

FIG. 32 is a graph illustrating a method of calculating respiratory rate and breathing timing information among biometric information according to an embodiment;

FIG. 33 is a diagram illustrating breathing timing information fed back to a user behavior guide program screen so that breathing timing can be induced during a meditation period or a mental and physical stabilization period;

FIG. 34 is a graph illustrating a method for calculating information on blood vessel elasticity among biometric information according to one embodiment;

FIG. 35 is a graph illustrating a method for calculating information on cardiovascular disease among biometric information according to one embodiment; and

FIG. 36 is a graph illustrating a method for calculating information on oxygen saturation among biometric information according to one embodiment.

## DETAILED DESCRIPTION OF THE EMBODIMENTS

The present disclosure will now be described more fully hereinafter with reference to the accompanying drawings, in which embodiments of the disclosure are shown. This disclosure may, however, be embodied in different forms and should not be construed as limited to the embodiments set forth herein.

It will also be understood that when a layer is referred to as being "on" another layer or substrate, it can be directly on the other layer or substrate, or intervening layers may also be present. The same reference numbers may indicate the same components throughout the specification.

It will be understood that, although the terms "first," "second," etc. may be used herein to describe various elements, these elements should not be limited by these terms. These terms are merely used to distinguish one element from another element. For instance, a first element discussed below could be termed a second element. Similarly, the second element could also be termed the first element.

Each of the features of the various embodiments of the present disclosure may be combined or combined with each other, in part or in whole, and technically various interlocking and driving are possible. Each embodiment may be implemented independently of each other or may be implemented together in an association.

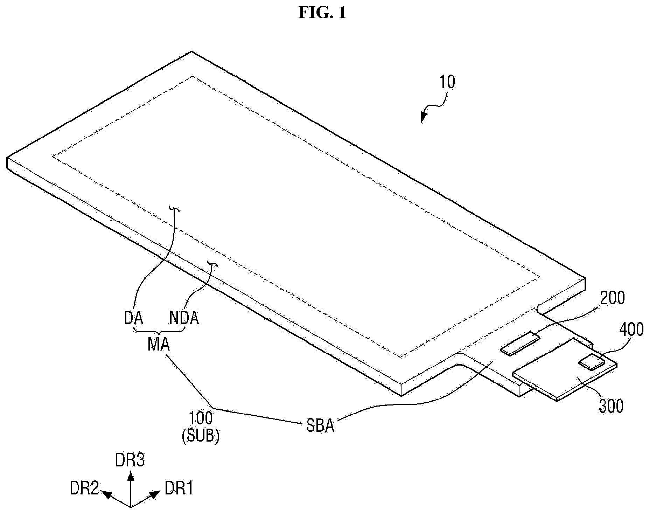

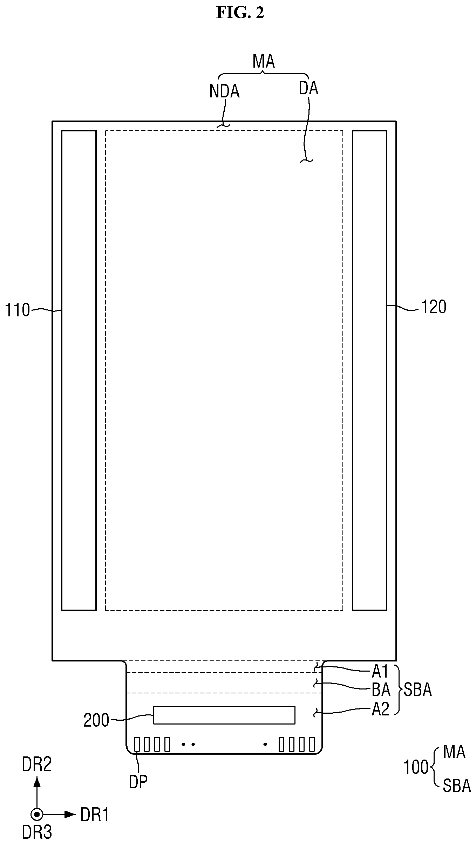

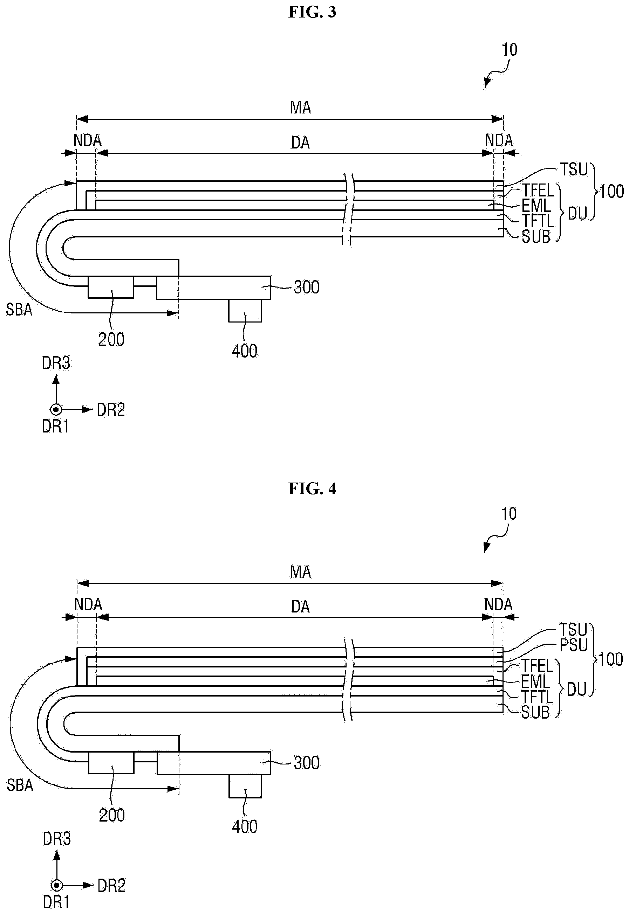

FIG. 1 is a perspective view illustrating a display device according to one embodiment. FIG. 2 is a plan view illustrating an arrangement structure of a display panel and a display driving circuit shown in FIG. 1. FIG. 3 is a side view showing the configuration of the display device shown in FIG. 1.

First, referring to FIGS. 1 and 2, a display device 10 according to one embodiment may be applied to portable electronic devices such as a mobile phone, a smartphone, a tablet personal computer (PC), a mobile communication terminal, an electronic organizer, an electronic book, a portable multimedia player (PMP), a navigation system, an ultra mobile PC (UMPC) or the like. Further, the display device 10 according to one embodiment may be applied as a display unit of a television, a laptop, a monitor, a billboard, or an Internet-of-Things (IoT) terminal. Alternatively, the display device 10 according to one embodiment may be applied to wearable devices such as a smart watch, a watch phone, a glasses type display, or a head mounted display (HMD). Further, the display device 10 according to one embodiment may be applied to a dashboard of a vehicle, a center fascia of a vehicle, a center information display (CID) disposed on a dashboard of a vehicle, a room mirror display in place of side mirrors of a vehicle, or a display disposed on a rear surface of a front seat for rear seat entertainment in a vehicle.

The display device 10 may be a light emitting display device such as an organic light emitting display device using an organic light emitting diode, a quantum dot light emitting display including a quantum dot light emitting layer, an inorganic light emitting display including an inorganic semiconductor, and a micro light emitting display using a micro or nano light emitting diode (LED). In the following description, it is assumed that the display device 10 is an organic light emitting display device, but the present disclosure is not limited thereto.

Referring to FIGS. 1 and 3, the display device 10 includes a display panel 100, a main driving circuit 200, a touch sensing unit TSU, a pressure sensing unit PSU (see FIG. 4), a circuit board 300, and a touch driving circuit 400.

The display panel 100 may, in a plan view, be formed in a rectangular shape having short sides in a first direction DR1 and long sides in a second direction DR2 crossing the first direction DR1. A corner where the short side in the first direction DR1 and the long side in the second direction DR2 meet may be right-angled or rounded to have a predetermined curvature. The planar shape of the display panel 100 is not limited to the rectangular shape, and may be formed in another polygonal shape, a circular shape or an elliptical shape. The display panel 100 may be formed to be flat, but is not limited thereto. For example, the display panel 100 may include a curved portion formed at left and right ends and having a constant curvature or a varying curvature. In addition, the display panel 100 may be formed flexibly so that it can be curved, bent, folded, or rolled.

A substrate SUB of the display panel 100 may include a main region MA and a sub-region SBA.

The main region MA may include a display area DA for displaying an image and a non-display area NDA that is a peripheral area of the display area DA.

The non-display area NDA may be disposed adjacent to the display area DA. The non-display area NDA may be an area outside the display area DA. The non-display area NDA may be disposed to surround the display area DA. The non-display area NDA may be an edge area of the display panel 100.

The display area DA includes display pixels for displaying an image, and light sensing pixels for sensing light reflected from a user's body part such as a face or a finger. Further, the display area DA may further include infrared light emitting pixels for emitting infrared light.

The display area DA may occupy most of the main region MA. The display area DA may be disposed at the center of the main region MA.

The display area DA may be divided into an image display area IDA (see FIG. 2) in which only the display pixels are disposed without the light sensing pixels, and a biometric information measurement area FSA (see FIG. 2) in which both the display pixels and the light sensing pixels are disposed. In other words, the light sensing pixels that are used to detect light incident or reflected from the front surface may be disposed together with the display pixels only in a predetermined part of the biometric information measurement area FSA in the display area DA of the display panel 100. An example in which the display pixels and the light sensing pixels are alternately arranged in the display area DA will be described below.

Referring to FIGS. 2 and 3, the sub-region SBA may protrude from one side of the main region MA in the second direction DR2. The length of the sub-region SBA in the second direction DR2 may be less than the length of the main region MA in the second direction DR2. The length of the sub-region SBA in the first direction DR1 may be substantially equal to or less than the length of the main region MA in the first direction DR1.

The sub-region SBA may include a first region A1, a second region A2, and a bending area BA.

The first region A1 is a region protruding from one side of the main region MA in the second direction DR2.

One side of the first region A1 may be in contact with the non-display area NDA of the main region MA, and the other side of the first region A1 may be in contact with the bending area BA. In other words, the first region A1 may be disposed between the non-display area NDA and the bending area BA.

The second region A2 is an region on which pads DP and the main driving circuit 200 are disposed. The main driving circuit 200 may be attached to driving pads of the second region A2 using a conductive adhesive member such as an anisotropic conductive layer. The circuit board 300 may be attached to the pads DP of the second region A2 using a conductive adhesive member. One side of the second region A2 may be in contact with the bending area BA.

The bending area BA is an area that can be bent. When the bending area BA is bent, the second region A2 may be disposed under the first region A1 and under the main region MA. The bending area BA may be disposed between the first region A1 and the second region A2. One side of the bending area BA may be in contact with the first region A1, and the other side of the bending area BA may be in contact with the second region A2.

As shown in FIG. 3, the sub-region SBA may be bent, and in this case, it may be disposed under the main region MA. The sub-region SBA may overlap the main region MA in a third direction DR3.

The touch sensing unit TSU for sensing a body part such as a finger, an electronic pen, or the like is formed or disposed on the front portion of the display panel 100. The touch sensing unit TSU may include a plurality of touch electrodes to sense a user's touch in a capacitive manner.

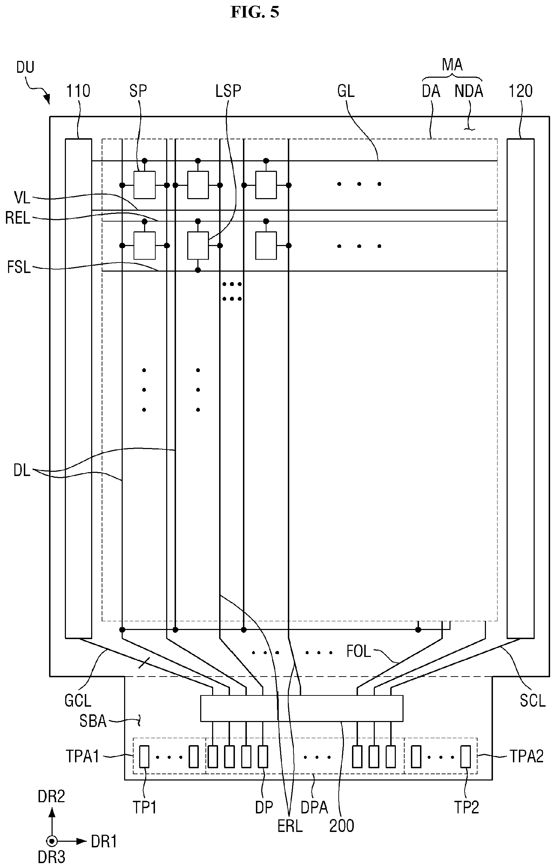

The touch sensing unit TSU includes a plurality of touch electrodes arranged to intersect each other in the first and second directions DR1 and DR2. For example, the plurality of touch electrodes include a plurality of driving electrodes arranged to be spaced apart from each other in parallel in the first direction DR1, and a plurality of sensing electrodes arranged to be spaced apart from each other in parallel in the second direction DR2 to intersect the plurality of driving electrodes with an organic material layer or an inorganic material layer interposed therebetween. The plurality of driving electrodes and the plurality of sensing electrodes may be formed to extend to a wiring region between display pixels SP (see FIG. 5) and light sensing pixels LSP (see FIG. 5) arranged in the display area DA so as not to overlap the display pixels SP (see FIG. 5) and the light sensing pixels LSP (see FIG. 5). The plurality of driving electrodes and the plurality of sensing electrodes form a mutual capacitance, and transmit touch sensing signals that vary according to a user's touch to the touch driving circuit 400.

The touch driving circuit 400 supplies touch driving signals to the plurality of driving electrodes and receives the touch sensing signals from the plurality of sensing electrodes. Then, the change in the mutual capacitance between the driving electrodes and the sensing electrodes is sensed according to the change in the magnitude of the touch sensing signal. The touch driving circuit 400 generates touch data according to the change in the mutual capacitance between the driving electrodes and the sensing electrodes and obtains a position where a touch is sensed. Accordingly, coordinate data of the position where the touch is sensed may be supplied to the main driving circuit 200.

FIG. 4 is a side view of another embodiment illustrating the configuration of the display device shown in FIG. 1. Like the embodiment of FIG. 3, in FIG. 4 there is provided a display module DU that includes the substrate SUB as well as a thin film transistor layer TFTL, an emission layer EML and an encapsulation layer TFEL.

The pressure sensing unit PSU for sensing the pressure applied by a body part such as a finger or the like may be disposed or formed on the front surface of the display panel 100, for example, on the surface between the display panel 100 and the touch sensing unit TSU. The pressure sensing unit PSU may be formed on the rear surface of the substrate SUB, in another embodiment.

The pressure sensing unit PSU is required in the case of detecting absolute blood pressure-related measurement values, but may not be required in the case of detecting relative blood pressure-related measurement values. Therefore, as shown in FIG. 3, the pressure sensing unit PSU may not be formed.

In the case where the pressure sensing unit PSU is formed, the pressure sensing unit PSU may be formed of a transparent sheet type in which a plurality of transparent electrodes are arranged in vertical and horizontal directions, and may be disposed on the front surface of the main area MA. Alternatively, the pressure sensing unit PSU may be disposed or formed inside or on the front portion of the display panel 100.

The pressure sensing unit PSU includes a plurality of pressure sensing electrodes arranged to intersect each other in the first direction DR1 and the second direction DR2. The plurality of pressure sensing electrodes include a plurality of lower electrodes arranged to be spaced apart from each other in parallel in the first direction DR1, and a plurality of upper electrodes arranged to be spaced apart from each other in parallel in the second direction DR2 to intersect the plurality of lower electrodes with a transparent inorganic (or organic) material layer interposed therebetween. The plurality of lower electrodes and the plurality of upper electrodes form a self-capacitance with a transparent inorganic (or organic) material layer interposed therebetween, and transmit pressure sensing signals that vary according to a user's touch pressure to the touch driving circuit 400.

When the pressure sensing unit PSU is disposed on the front surface of the display panel 100, the pressure sensing electrodes, e.g., the plurality of lower electrodes and the plurality of upper electrodes, of the pressure sensing unit PSU may be formed to extend to the wiring region between the display pixels and the light sensing pixels arranged in the display area DA so as not to overlap the display pixels SP (see FIG. 5) and the light sensing pixels LSP (see FIG.

5). Additionally, the touch driving circuit 400 may supply a reference voltage to the lower electrodes of the pressure sensing unit PSU, and receive pressure sensing signals from the upper electrodes thereof, thereby sensing the changes in the self-capacitance of the pressed areas using the pressure sensing signals. Accordingly, the touch driving circuit 400 may generate pressure data according to the amount of change in the self-capacitance and sensing coordinate data of a position where a touch is sensed and supply the generated data to the main driving circuit 200. The pressure sensing unit PSU may be applied to various other structures in addition to the structure using the pressure sensing electrodes, and is not limited to the description of FIGS. 3 and 4.

The circuit board 300 may be attached to one end of the sub-region SBA. Thus, the circuit board 300 may be electrically connected to the display panel 100 and the main driving circuit 200. The display panel 100 and the main driving circuit 200 may receive digital video data, timing signals, and driving voltages through the circuit board 300. The circuit board 300 may be a flexible printed circuit board, a printed circuit board, or a flexible film such as a chip on film.

The main driving circuit 200 may generate electrical signals such as data voltages and control signals for driving the display panel 100. The touch driving circuit 400 including the main driving circuit 200 may be formed as an integrated circuit (IC) and attached onto the display panel 100 or the circuit board 300 by a chip on glass (COG) method, a chip on plastic (COP) method, or an ultrasonic bonding method, but the present disclosure is not limited thereto. For example, the touch driving circuit 400 including the main driving circuit 200 may be attached onto the circuit board 300 by a chip on film (COF) method.

FIG. 5 is a layout diagram schematically showing an example of the display panel illustrated in FIGS. 1 to 4. For example, FIG. 5 is a layout diagram illustrating the display area DA and the non-display area NDA of a display module DU before the touch sensing unit TSU is formed thereon.

Referring to FIG. 5 together with FIG. 4, a display scan driver 110, a light sensing scan driver 120, and a main driving circuit 200 may be disposed on the display panel 100 of the display device 10 according to one embodiment. Further, a touch driving circuit 400 and a power supply unit may be disposed on the circuit board 300 connected to the display panel 100. Here, the main driving circuit 200 and the touch driving circuit 400 may be integrally formed of a one-chip type, and may be mounted onto the display panel 100 or the circuit board 300. However, hereinafter, for simplicity of functional description, an example in which the main driving circuit 200 and the touch driving circuit 400 are formed as different integrated circuits will be described.

Referring to FIG. 5, the display panel 100 may include the display pixels SP, the light sensing pixels LSP, display scan lines GL, emission control lines VL, data lines DL, sensing scan lines FSL, sensing reset lines REL, and light sensing lines ERL that are disposed in the display area DA. Each of the display scan driver 110 and the light sensing scan driver 120 are disposed in the non-display area NDA.

The display scan lines GL sequentially supply the display scan signals applied in units of horizontal lines from the display scan driver 110 to the display pixels SP and light sensing pixels LSP for each horizontal line. The display scan lines GL may extend in the first direction DR1 and may be spaced apart from each other in the second direction DR2 intersecting the first direction DR1.

The emission control lines VL sequentially supply the emission control signals applied in units of horizontal lines from the display scan driver 110 to the display pixels SP and the light sensing pixels LSP for each horizontal line. The emission control lines VL may extend in the first direction DR1 to be in parallel with the display scan lines GL, and may be spaced apart from each other in the second direction DR2 intersecting the first direction DR1.

The data lines DL may supply the data voltage received from the main driving circuit 200 to the plurality of display pixels SP. The plurality of data lines DL may extend in the second direction DR2 and may be spaced apart from each other in the first direction DR1.

The light sensing scan lines FSL sequentially supply the sensing scan signals applied in units of horizontal lines from the light sensing scan driver 120 to the plurality of light sensing pixels LSP. The light sensing scan line FSL may extend in the first direction DR1 and may be spaced apart from each other in the second direction DR2 intersecting the first direction DR1.

The sensing reset lines REL sequentially supply the sensing reset signals applied in units of horizontal lines from the light sensing scan driver 120 to the plurality of light sensing pixels LSP for each horizontal line. The sensing reset lines REL may extend in the first direction DR1 to be in parallel with the light sensing scan lines FSL, and may be spaced apart from each other in the second direction DR2 intersecting the first direction DR1.

The light sensing lines ERL are connected between the light sensing pixels LSP and the main driving circuit 200 to supply the light sensing signals outputted from the light sensing pixels LSP to the main driving circuit 200. The light sensing lines ERL may be disposed and extended in the second direction DR2 according to the arrangement direction of the main driving circuit 200, and may be spaced apart from each other in the first direction DR1.

The non-display area NDA may surround the display area DA. The non-display area NDA may include the display scan driver 110, the light sensing scan driver 120, fan-out lines FOL, gate control lines GCL, and light sensing control lines SCL.

The display pixels SP and the light sensing pixels LSP may form a first unit pixel and may be arranged in a matrix form in the first direction DR1 and the second direction DR2 in the display area DA. When at least one infrared light emitting pixel is additionally disposed in the display area DA, the display pixels SP and at least one infrared light emitting pixel may form a second unit pixel, and the second unit pixels and the first unit pixels may be alternately arranged in a matrix form in the display area DA.

For example, three display pixels SP that respectively display red, green, and blue light, and one light sensing pixel LSP may form one first unit pixel. In this case, four pixels may form one first unit pixel. In addition, three display pixels SP that respectively display red, green, and blue light, and one infrared light emitting pixel may form one second unit pixel. In this case, four pixels may form one second unit pixel. The first unit pixels and the second unit pixels may be alternately arranged in horizontal or vertical stripes in a matrix form. Alternatively, the first unit pixels and the second unit pixels may be alternately arranged in a zigzag shape in a plan view, and may be arranged in a matrix form in one diagonal direction.

Each of the red, green, and blue display pixels SP and the infrared light emitting pixels may be connected to any one of the display scan lines GL and any one of the emission control lines VL. During an image display period, the red, green, and blue display pixels SP may receive the data voltage of the data line DL according to the display scan signal of the display scan line GL and the emission control signal of the emission control line VL, and may supply a driving current to the light emitting element according to the data voltage, thereby emitting light. Here, during the measurement period of biometric information such as blood pressure, heart rate, oxygen saturation, blood vessel elasticity, and the like, the display pixels SP displaying at least one color among the red, green, and blue display pixels SP may selectively receive the data voltage for light emission together with the display scan signal and the emission control signal and display light. In other words, the display pixels SP, which exhibit at least one color among the red, green, and blue display pixels SP, may selectively receive the data voltage for light emission in conjunction with the display scan signal and the emission control signal, subsequently producing light. Further, during the measurement period of biometric information such as blood pressure, heart rate, and the like, the infrared light emitting pixels may selectively receive the data voltage for light emission together with the display scan signal and the emission control signal, and consequently emit infrared light.

The light sensing pixels LSP may be alternately arranged with the red, green, and blue display pixels SP in a vertical or horizontal direction. Each of the light sensing pixels LSP may be connected to one of the light sensing scan lines FSL, one of the sensing reset lines REL, and one of the light sensing lines ERL. During the measurement period of biometric information such as blood pressure, respiratory rate, oxygen saturation, or the presence or absence of cardiovascular disease, each of the light sensing pixels LSP is reset in response to the sensing reset signal from the sensing reset lines REL. Subsequently, they may generate the light sensing signal corresponding to the amount of light reflected and incident from the front surface. Further, each of the light sensing pixels LSP may transmit the light sensing signal to the light sensing line ERL in response to the sensing scan signal from the light sensing scan lines FSL.

Alternatively, each of the light sensing pixels LSP may be connected to one of the display scan lines GL in units of horizontal lines. Each of the light sensing pixels LSP may generate the light sensing signal corresponding to the amount of light reflected and incident from the front surface, and may output the light sensing signal to the light sensing line ERL in response to the display scan signal inputted through the display scan line GL.

The display scan driver 110 may be provided in the non-display area NDA. Although the display scan driver 110 is illustrated to be disposed on one side (e.g., left side) of the display panel 100, it is not limited to that shown in the drawings herein. For example, the display scan driver 110 may be disposed on both sides (e.g., left and right sides) of the display panel 100.

The display scan driver 110 may be electrically connected to the main driving circuit 200 through the gate control lines GCL. The display scan driver 110 receives the scan control signal from the main driving circuit 200, and sequentially generates the display scan signals in units of horizontal line driving periods according to the scan control signal and sequentially supplies them to the display scan lines GL. Further, the display scan driver 110 may sequentially generate the emission control signals according to the scan control signal from the main driving circuit 200 and sequentially supply them to the emission control lines VL.

The gate control lines GCL may extend from the main driving circuit 200 to the display scan driver 110 according to the arrangement position of the display scan driver 110. The gate control lines GCL may supply the scan control signals received from the main driving circuit 200 to the display scan driver 110.

The light sensing scan driver 120 may be disposed in another non-display area NDA different from that of the display scan driver 110. FIG. 5 illustrates that the light sensing scan driver 120 is disposed on the other side (for example, the right side) of the display panel 100, but the present disclosure is not limited thereto. The light sensing scan driver 120 may be electrically connected to the main driving circuit 200 through the light sensing control lines SCL. The light sensing scan driver 120 receives the light sensing control signal from the main driving circuit 200, and sequentially generates the reset control signals and the light sensing scan signals in units of horizontal line driving periods according to the light sensing control signal. Then, the sequentially generated reset control signals are sequentially supplied to the sensing reset lines REL. Further, the light sensing scan driver 120 may sequentially generate the sensing scan signals according to the light sensing control signal from the main driving circuit 200 and sequentially supply them to the sensing scan lines FSL.

The light sensing control lines SCL may extend from the main driving circuit 200 to the light sensing scan driver 120 according to the arrangement position of the light sensing scan driver 120. The light sensing control lines SCL may supply the light sensing control signals received from the main driving circuit 200 to the light sensing scan driver 120.

The sub-region SBA may include the main driving circuit 200, a display pad area DPA, and first and second touch pad areas TPA1 and TPA2. The display pad area DPA, the first touch pad area TPA1, and the second touch pad area TPA2 may be disposed at the edge of the sub-region SBA. The display pad area DPA, the first touch pad area TPA1, and the second touch pad area TPA2 may be electrically connected to the circuit board 300 by using an anisotropic conductive layer or a low-resistance high-reliability material such as SAP. The first touch pad area TPA1 may include a plurality of first touch pads TP1 and the second touch pad area TPA2 may include a plurality of second touch pads TP2.

The fan-out lines FOL may extend from the main driving circuit 200 to the display area DA. Further, the fan-out lines FOL are connected such that the data voltage received from the main driving circuit 200 may be supplied to each of the plurality of data lines DL.

The main driving circuit 200 may output signals and voltages for driving the display panel 100 to the fan-out lines FOL. The main driving circuit 200 may supply a data voltage to the data line DL through the fan-out lines FOL. The data voltage may be supplied to the plurality of display pixels SP to determine the luminance of the display pixels SP. The main driving circuit 200 may supply the scan control signal to the display scan driver 110 through the gate control line GCL.

The main driving circuit 200 receives the light sensing signals from the light sensing pixels LSP through the light sensing lines ERL, and detects a photoplethysmography signal among biometric signals corresponding to the change in the magnitudes of the light sensing signals, in other words, pulse wave signals.

In addition to the pulse wave signal, the biometric signals may further include an EMG signal, an EEG signal, and the like. However, an example in which the main driving circuit 200 detects and analyzes pulse wave signals among biometric signals to measure the user's biometric information will be described below. The biometric information of the user includes information such as blood pressure, heart rate, heart rate variability, respiratory rate, blood vessel elasticity, the occurrence or non-occurrence of cardiovascular disease, oxygen saturation, and the like.

The main driving circuit 200 may guide a pulse wave signal detection process with a preset application program screen so that the user's pulse wave signals may be accurately detected, and may analyze the pulse wave signals to sample and select more accurately detected pulse wave signals. In other words, the main driving circuit 200 can guide a pulse wave signal detection process through a preset application program screen, enabling the precise detection of the user's pulse wave signals. It can then analyze the pulse wave signals to sample and select the pulse wave signals that have been detected with greater accuracy. Then, the main driving circuit 200 analyzes the pulse wave signals in units of preset periods to measure biometric information such as blood pressure, heart rate, heart rate variability, respiratory rate, blood vessel elasticity, the occurrence or non-occurrence of cardiovascular disease, oxygen saturation, and the like. Accordingly, the main driving circuit 200 may display the measurement results of the biometric information such as blood pressure, heart rate, heart rate variability, respiratory rate, blood vessel elasticity, the occurrence or non-occurrence of cardiovascular disease, oxygen saturation, and the like on the application program screen.

Additionally, the main driving circuit 200 may utilize a user behavior guide program designed to promote both mental and physical stabilization. This program displays behavior guidance information on its screen, providing the user with time for mental and physical stabilization as well as meditation. Additionally, the program can integrate sound and vibration features related to meditation for an enhanced experience.

The user behavior guide program is a preset application program that users can access for meditation and mental and physical stabilization. The program displays images on the screen to assist mental and physical stabilization or meditation and supports sound and vibration functions that complement the images. Hereinafter, the term "a preset user behavior guidance program screen" refers to images on the screen of the preset user behavior guidance program.

The user may selectively execute the user behavior guide program, which is an application program, and perform meditation by referring to the user behavior guidance information. Additionally, the user may measure biometric information in real time by simultaneously executing a biometric information measuring program linked to the behavior guide program. To accomplish this, the main driving circuit 200 displays at least one of biometric information detected in real time, for example, heart rate variability, respiratory rate, and breathing timing information on the application screen as well as audio. At the same time, the user behavior guidance information image and sound are displayed so that the user can adjust the respiratory rate and breathing timing during the biometric information measurement period and the meditation period. Accordingly, the user can make additional efforts to mentally and physically stabilize themselves and adjust their respiratory rate and breathing timing by referring to the information displayed on the user behavior guide screen and sound and vibration cues.

On the other hand, the main driving circuit 200 may generate digital video data corresponding to touch coordinates according to touch coordinate data from the touch driving circuit 400, or may execute an application indicated by an icon displayed on the user's touch coordinates.

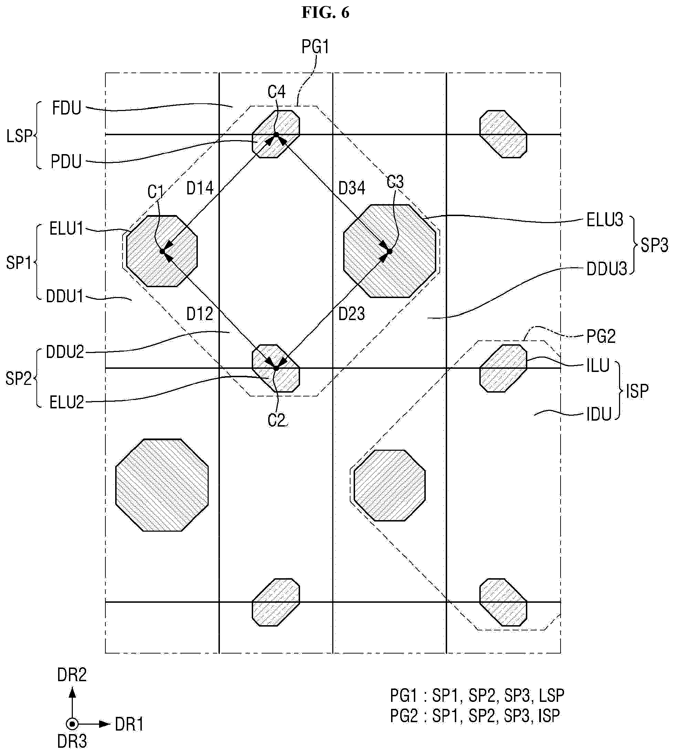

FIG. 6 is a layout diagram illustrating a display area according to one embodiment.

Referring to FIG. 6, the display area DA may include the display pixels SP, infrared light emitting pixels ISP, and the light sensing pixels LSP. Here, the display pixels SP may be divided into the first display pixels SP1, the second display pixels SP2, and the third display pixels SP3.

The light sensing pixel LSP, the first display pixel SP1, the second display pixel SP2, and the third display pixel SP3 may be referred to as a first unit pixel PG1. Further, the infrared light emitting pixel ISP, the first display pixel SP1, the second display pixel SP2, and the third display pixel SP3 may be referred to as a second unit pixel PG2.

The first and second unit pixels PG1 and PG2 may be a minimum unit display pixels capable of displaying white, and each first unit pixel PG1 may sense light. The first unit pixels PG1 and the second unit pixels PG2 may be alternately arranged in a zigzag shape in a plan view, and may be arranged in a matrix form in one diagonal direction. Further, the first unit pixels PG1 and the second unit pixels PG2 may be alternately arranged in horizontal or vertical stripes in a matrix form in a plan view.

The first display pixel SP1 may include a first light emitting portion ELU1 that emits first light, and a first pixel driver DDU1 for applying a driving current to the light emitting element of the first light emitting portion ELU1. The first light may be light of a red wavelength band. For example, the main peak wavelength of the first light may be located at approximately 600 nm to 750 nm.

The second display pixel SP2 may include a second light emitting portion ELU2 that emits second light, and a second pixel driver DDU2 for applying a driving current to the light emitting element of the second light emitting portion ELU2. The second light may be light of a blue wavelength band. For example, the main peak wavelength of the second light may be located at approximately 370 nm to 460 nm.

The third display pixel SP3 may include a third light emitting portion ELU3 that emits third light, and a third pixel driver DDU3 for applying a driving current to the light emitting element of the third light emitting portion ELU3. For example, the third light may be light of a green wavelength band. For example, the main peak wavelength of the third light may be located at approximately 480 nm to 560 nm.

The infrared light emitting pixel ISP may include an infrared light emitting portion ILU emitting light of an infrared wavelength band and an infrared light pixel driver IDU for applying a driving current to the light emitting element of the infrared light emitting portion ILU. The main peak wavelength of the infrared light may be located at approximately 750 nm to 1 mm.

The light sensing pixel LSP includes a light sensing portion PDU and a sensing driver FDU.

In the first unit pixel GP1, the first to third pixel drivers DDU1 to DDU3 may be arranged in a preset order in the first direction DR1. Alternatively, any one of the first to third pixel drivers DDU1 to DDU3 may be disposed in the first direction DR1 of another adjacent pixel driver. Further, the sensing driver FDU may be disposed in the first direction DR1 of any one of the first to third pixel drivers DDU1 to DDU3. Alternatively, the sensing driver FDU may be disposed in the second direction DR2 of any one of the first to third pixel drivers DDU1 to DDU3.

The first pixel drivers DDU1 adjacent to each other in the data line DL direction may be disposed in the second direction DR2. The second pixel drivers DDU2 adjacent to each other in the direction of the data line DL may be disposed in the second direction DR2. Similarly, all the sensing drivers FDU adjacent to each other in the direction of the data line DL may also be disposed in the second direction DR2.

The first light emitting portion ELU1, the second light emitting portion ELU2, the third light emitting portion ELU3, the infrared light emitting portion ILU, and the light sensing portion PDU may have a rectangular, octagonal, or rhombic planar shape, but the present disclosure is not limited thereto. The first light emitting portion ELU1, the second light emitting portion ELU2, the third light emitting portion ELU3, the infrared light emitting portion ILU, and the light sensing portion PDU may have another polygonal planar shape other than a rectangle, an octagon, and a rhombus.

On the other hand, due to the arrangement position and planar shape of the first light emitting portion ELU1, the second light emitting portion ELU2, the third light emitting portion ELU3, and the light sensing portion PDU, a distance D12 between a center C1 of the first light emitting portion ELU1 and a center C2 of the second light emitting portion ELU2 adjacent to each other, a distance D23 between the center C2 of the second light emitting portion ELU2 and a center C3 of the third light emitting portion ELU3 adjacent to each other, a distance D14 between the center C1 of the first light emitting unit ELU1 and a center C2 of the second light emitting portion ELU2 adjacent to each other in another direction, and a distance D34 between a center C4 of the second light emitting portion ELU2 and the center C3 of the third light emitting portion ELU3 may be substantially the same.

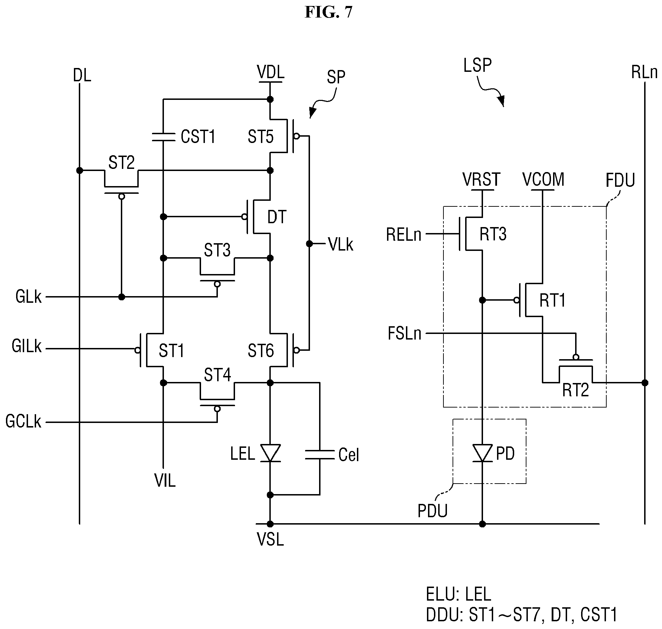

FIG. 7 is a circuit diagram illustrating a display pixel and a light sensing pixel according to one embodiment.

Referring to FIG. 7, each display pixel SP according to one embodiment may be connected to a $k^{th}$ display initialization line GILk, a $k^{th}$ display scan line GLk, a $k^{th}$ display control line GCLk, and a $k^{th}$ emission control line VLk. In addition, the display pixel SP may be connected to a first driving voltage line VDL to which a first driving voltage is supplied, a second driving voltage line VSL to which a second driving voltage is supplied, and a third driving voltage line VIL to which a third driving voltage is supplied. Hereinafter, k, n and the like may be used in place of numbers are positive integers excluding 0.

The display pixel SP may include a light emitting portion ELU and a pixel driver DDU. The light emitting portion ELU may include a light emitting element LEL. The pixel driver DDU may include a driving transistor DT, switch elements, and a capacitor CST1. The switch elements include the first to sixth transistors ST1, ST2, ST3, ST4, ST5, and ST6.

The driving transistor DT may include a gate electrode, a first electrode, and a second electrode. The driving transistor DT controls a drain-source current Ids (hereinafter, referred to as "driving current") flowing between the first electrode and the second electrode according to a data voltage applied to the gate electrode. The driving current Ids flowing through a channel of the driving transistor DT is proportional to the square of the difference between a threshold voltage and a voltage Vsg between the first electrode and the gate electrode of the driving transistor DT, as shown in Eq. (1).

$$Ids = k' \times (Vsg\text{-}Vth)^2 \quad \text{................................(1)}$$

In Eq. (1), k' is a proportional coefficient determined by the structure and physical characteristics of the driving transistor DT, Vsg is a voltage between the first electrode and the gate electrode of the driving transistor DT, and Vth is a threshold voltage of the driving transistor DT.

The light emitting element LEL emits light by the driving current Ids. As the driving current Ids increases, the amount of light emitted from the light emitting element LEL may increase.

The light emitting element LEL may be an organic light emitting diode including an organic light emitting layer disposed between an anode electrode and a cathode electrode. Alternatively, the light emitting element LEL may be an inorganic light emitting element including an inorganic semiconductor disposed between an anode electrode and a cathode electrode. Alternatively, the light emitting element LEL may be a quantum dot light emitting element including a quantum dot light emitting layer disposed between an anode electrode and a cathode electrode. Alternatively, the light emitting element LEL may be a micro light emitting element including a micro light emitting diode disposed between an anode electrode and a cathode electrode.

The anode electrode of the light emitting element LEL may be connected to a first electrode of the fourth transistor ST4 and a second electrode of the sixth transistor ST6, and the cathode electrode of the light emitting element LEL may be connected to the second driving voltage line VSL. A parasitic capacitance Cel may be formed between the anode electrode and the cathode electrode of the light emitting element LEL.

The first transistor ST1 is turned on by a display initialization signal of the k$^{th}$ display initialization line GILk to connect the gate electrode of the driving transistor DT to the third driving voltage line VIL. Accordingly, the third driving voltage VINT of the third driving voltage line VIL may be applied to the gate electrode of the driving transistor DT. The gate electrode of the first transistor ST1 may be connected to the k$^{th}$ display initialization line GILk, the first electrode of the first transistor ST1 may be connected to the gate electrode of the driving transistor DT, and the second electrode of the first transistor ST1 may be connected to the third driving voltage line VIL.

The second transistor ST2 is turned on by a display scan signal of the k$^{th}$ display scan line GLk to connect the first electrode of the driving transistor DT to the data line DL. Accordingly, the data voltage of the data line DL may be applied to the first electrode of the driving transistor DT. The gate electrode of the second transistor ST2 may be connected to the k$^{th}$ display scan line GLk, the first electrode of the second transistor ST2 may be connected to the first electrode of the driving transistor DT, and the second electrode of the second transistor ST2 may be connected to the data line DL.

The third transistor ST3 is turned on by the display scan signal of the k$^{th}$ display scan line GLk to connect the gate electrode of the driving transistor DT to the second electrode of the third transistor ST3. When the gate electrode of the driving transistor DT is connected to the second electrode of the third transistor ST3, the driving transistor DT is driven as a diode. The gate electrode of the third transistor ST3 may be connected to the k$^{th}$ display scan line GLk, the first electrode of the third transistor ST3 may be connected to the second electrode of the driving transistor DT, and the second electrode of the third transistor ST3 may be connected to the gate electrode of the driving transistor DT.

The fourth transistor ST4 is turned on by a display control signal of the k$^{th}$ display control line GCLk to connect the anode electrode of the light emitting element LEL to the third driving voltage line VIL. The third driving voltage of the third driving voltage line VIL may be applied to the anode electrode of the light emitting element LEL. The gate electrode of the fourth transistor ST4 is connected to the k$^{th}$ display control line GCLk, the first electrode of the fourth transistor ST4 is connected to the anode electrode of the light emitting element LEL, and the second electrode of the fourth transistor ST4 is connected to the third driving voltage line VIL.

The fifth transistor ST5 is turned on by an emission signal of a k$^{th}$ emission control line VLk to connect the first electrode of the driving transistor DT to the first driving voltage line VDL. The gate electrode of the fifth transistor ST5 is connected to the k$^{th}$ emission control line VLk, the first electrode of the fifth transistor ST5 is connected to the first driving voltage line VDL, and the second electrode of the fifth transistor ST5 is connected to the first electrode of the driving transistor DT.

The sixth transistor ST6 is disposed between the second electrode of the driving transistor DT and the anode electrode of the light emitting element LEL. The sixth transistor ST6 is turned on by the emission control signal of the $k^{th}$ emission control line VLk to connect the second electrode of the driving transistor DT to the anode electrode of the light emitting element LEL. The gate electrode of the sixth transistor ST6 is connected to the $k^{th}$ emission control line VLk, the first electrode of the sixth transistor ST6 is connected to the second electrode of the driving transistor DT, and the second electrode of the sixth transistor ST6 is connected to the anode electrode of the light emitting element LEL.

When both the fifth transistor ST5 and the sixth transistor ST6 are turned on, the driving current Ids of the driving transistor DT according to the data voltage applied to the gate electrode of the driving transistor DT may flow to the light emitting element LEL.

The capacitor CST1 is formed between the gate electrode of the driving transistor DT and the first driving voltage line VDL. The first capacitor electrode of the capacitor CST1 may be connected to the gate electrode of the driving transistor DT, and the second capacitor electrode of the capacitor CST1 may be connected to the first driving voltage line VDL.

When the first electrode of each of the driving transistor DT and the first to sixth transistors ST1 to ST6 is a source electrode, the second electrode of each of the driving transistor DT and the first to sixth transistors ST 1 to ST6 may be a drain electrode. Alternatively, when the first electrode of each of the driving transistor DT and the first to sixth transistors ST 1 to ST6 is a drain electrode, the second electrode of each of the driving transistor DT and the first to sixth transistors ST1 to ST6 may be a source electrode.

An active layer of each of the driving transistor DT and the first to sixth transistors ST 1 to ST6 may be formed of any one of polysilicon, amorphous silicon, or an oxide semiconductor. In FIG. 7, the first to sixth transistors ST1 to ST6, and the driving transistor DT have been mainly described as being formed of a P-type metal oxide semiconductor field effect transistor (MOSFET), but the present disclosure is not limited thereto. For example, the first to sixth transistors ST1 to ST6, and the driving transistor DT may be formed of an N-type MOSFET. Alternatively, at least one of the first to sixth transistors ST1 to ST6 may be formed of an N-type MOSFET.

The light sensing pixels LSP are respectively electrically connected to an $n^{th}$ sensing reset line RSLn, an $n^{th}$ light sensing scan line FSLn, and an $n^{th}$ light sensing line RLn. Each of the light sensing pixels LSP may be reset by a reset signal from the $n^{th}$ sensing reset line RSLn, and may transmit a light sensing signal to each $n^{th}$ light sensing line RLn in response to a sensing scan signal from the $n^{th}$ light sensing scan line FSLn.

The light sensing pixels LSP may be divided into the light sensing portion PDU including a light sensing element PD, and the sensing driver FDU including first to third sensing transistors RT1 to RT3 and a sensing capacitor. Here, the sensing capacitor may be formed in parallel with the light sensing element PD.

The first sensing transistor RT1 of the sensing driver FDU may allow a light sensing current to flow according to the voltages of the light sensing element PD and the sensing capacitor. The amount of the light sensing current may vary depending on a voltage applied to the light sensing element PD and the sensing capacitor. The gate electrode of the first sensing transistor RT1 may be connected to the second electrode of the light sensing element PD. The first electrode of the first sensing transistor RT1 may be connected to a common voltage source Vcom to which a common voltage is applied. The second electrode of the first sensing transistor RT1 may be connected to the first electrode of the second sensing transistor RT2.

When a sensing scan signal of a gate-on voltage is applied to the $n^{th}$ light sensing scan line FSLn, the second sensing transistor RT2 may allow a sensing current of the first sensing transistor RT1 to flow to the $n^{th}$ light sensing line RLn. In this case, the $n^{th}$ light sensing line RLn may be charged with a sensing voltage by the sensing current. The gate electrode of the second sensing transistor RT2 may be connected to the $n^{th}$ light sensing scan line FSLn, the first electrode of the second sensing transistor RT2 may be connected to the second electrode of the first sensing transistor RT1, and the second electrode of the second sensing transistor RT2 may be connected to the $n^{th}$ light sensing line RLn.

When a reset signal of the gate-on voltage is applied to the $n^{th}$ sensing reset line RSLn, the third sensing transistor RT3 may reset the voltages of the light sensing element PD and the sensing capacitor to a reset voltage of a reset voltage source VRST. The gate electrode of the third sensing transistor RT3 may be connected to the sensing reset line RSL, the first electrode of the third sensing transistor RT3 may be connected to the reset voltage source VRST, and the second electrode of the third sensing transistor RT3 may be connected to the second electrode of the light sensing element PD.

It is illustrated in FIG. 7 that the first sensing transistor RT1 and the second sensing transistor RT2 are formed of a P-type MOSFET, and the third sensing transistor RT3 is formed of an N-type MOSFET. However, an embodiment of the present disclosure is not limited thereto, and they may be selectively formed in the same type or different types. Further, any one of the first electrode and the second electrode of each of the first sensing transistor RT1, the second sensing transistor RT2, and the third sensing transistor RT3 may be the source electrode and the other one may be the drain electrode.

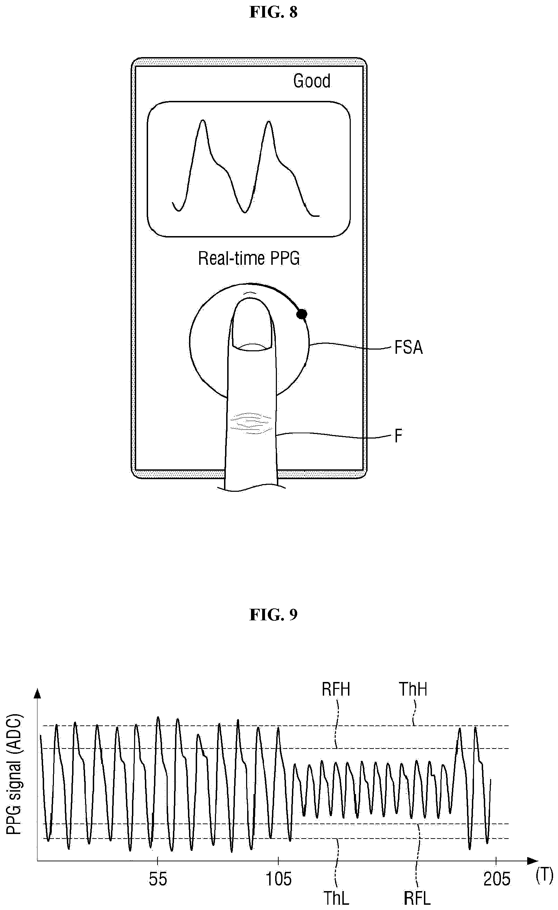

FIG. 8 is a diagram showing an image display screen and a body part touch area detection process during a biometric information measurement period.

Referring to FIG. 8, during the biometric information detection period, the main driving circuit 200 may play meditation music and show visuals through a preset user behavior guide program so that the user can have time for meditation or time for mental and physical stabilization. In addition, main driving circuit 200 may cause the data voltage to be supplied to the first and second unit pixels PG1 and PG2 of the display panel 100 based on the user's selection or control during meditation. Additionally, control signals may be supplied to the display scan driver 110 and the light sensing scan driver 120 to display a preset guide screen, which aids in the detection of pulse wave signals, within the display area DA. A part of the guide screen and audio may further display meditation sound and a separate meditation support video.

The main driving circuit 200 displays the biometric information measurement area FSA where a touch of a body part, such as a finger F or the like, is sensed on the guide screen for pulse wave signal detection, and displays the waveforms of the pulse wave signals detected in real time on the display window to guide the pulse wave signal detection process. At this time, the main driving circuit 200 displays a period required for biometric information measurement, a pulse wave signal detection period, and the biometric information measurement area FSA in the form of a circular or bar graph on the application program screen.

For example, during the meditation period and biometric information detection period, the main driving circuit 200 receives touch position coordinates sensed by the touch sensing unit TSU or the pressure sensing unit PSU using the touch driving circuit 400. Then, the main driving circuit 200 supplies the data voltage to the first and second unit pixels PG1 and PG2 arranged at the portion of the biometric information measurement area FSA that is touched by the finger F, and supplies the control signals to the display scan driver 110 and the light sensing scan driver 120. At this time, the main driving circuit 200 may supply a preset data voltage to at least one of the first and second display pixels SP1 and SP2 included in the first and second unit pixels PG1 and PG2, so that an optical signal may be detected by at least one of the green light or the red light. Thereafter, the main driving circuit 200 receives the optical signals, in other words, the light sensing signals, from the light sensing pixels LSP through the light sensing lines ERL of the display panel 100.

The main driving circuit 200 detects the pulse wave signals (PPG signals) corresponding to the light sensing signals received in real time and stores them as digital signal data. The pulse wave signals correspond to the magnitudes of the light sensing signals and the changes in those magnitudes. The main driving circuit 200 displays the pulse wave signals detected in real time on the display window of the application program screen in a graphic form of a graph type.

FIG. 9 is a graph showing a pulse wave signal detected in real time during a biometric information measurement period and an inaccurate pulse wave signal detection process.

Referring to FIG. 9, the main driving circuit 200 performs preset signal processing processes for light sensing signals, such as amplification, filtering, and sampling of the light sensing signals detected by the light sensing pixels LSP at a position where a body part, such as the finger F or the like, is touched. Then, the pulse wave signals PPG corresponding to the magnitudes of the signal-processed light sensing signals and the changes in those magnitudes are generated, stored in units of preset periods, and monitored.

Hereinafter, a high pulse may refer to a pulse that changes from the peak(high peak) of the rising state to the falling state and a low pulse may refer to a pulse that changes from the peak(low peak) of the falling state to the rising state.

The main driving circuit 200 analyzes the high pulse magnitude change and the low pulse magnitude change of the pulse wave signals PPG generated and stored in real time, and calculates an average magnitude value RFH of high pulses and an average magnitude value RFL of low pulses in real time. Then, the main driving circuit 200 sets a normal pulse wave signal detection period or an inaccurate pulse wave signal detection period in real time according to a comparison result of the average magnitude value RFH of high pulses to a preset high threshold ThH and the average magnitude value RFL of low pulses to a preset low threshold ThL.

For example, the main driving circuit 200 sets the normal pulse wave signal detection period when the average magnitude value RFH of high pulses is greater than or equal to the preset high threshold ThH during a plurality of preset frame periods. Further, the main driving circuit 200 may set the normal pulse wave signal detection period when the average magnitude value RFL of low pulses is greater than the preset low threshold ThL during a plurality of preset frame periods.

Alternatively, the main driving circuit 200 sets the inaccurate pulse wave signal detection period when the average magnitude value RFH of high pulses is smaller than the preset high threshold ThH during a plurality of preset frame periods. Further, the main driving circuit 200 may set the inaccurate pulse wave signal detection period when the average magnitude value RFL of low pulses is smaller than the preset low threshold ThL during a plurality of preset frame periods.

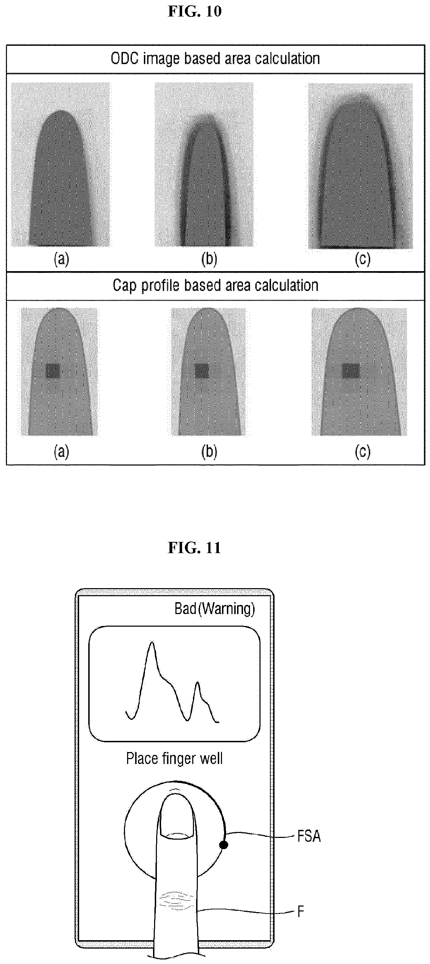

FIG. 10 is an image diagram showing a state in which a pulse wave signal is inaccurately detected during a biometric information measurement period.

Referring to FIG. 10, the main driving circuit 200 performs preset signal processing processes on light sensing signals detected by the light sensing pixels LSP of a position where a body part, such as the finger F or the like, is touched. Further, the main driving circuit 200 generates an image (ODC image) based on the signal value detected by the light sensing pixels LSP. Then, a touch area or a touch shape is analyzed based on the signal values and the image detected by the light sensing pixels LSP. As illustrated in image in FIG. 10B, when the touch area is smaller than a reference area, the inaccurate pulse wave signal detection period may be set.

Alternatively, the main driving circuit 200 may generate a capacitance (Cap) profile for signal values based on the signal values detected by the light sensing pixels LSP. Further, the main driving circuit 200 analyzes the touch area or the touch shape based on the signal values and the image detected by the light sensing pixels LSP. Then, the touch area or the touch shape is analyzed based on the Cap profile detected by the light sensing pixels LSP. As illustrated in image in FIG. 10B, when the touch area is smaller than the reference area, the inaccurate pulse wave signal detection period may be set.

FIG. 11 is a diagram showing an image display screen displayed when an inaccurate pulse wave signal is detected during a biometric information measurement period.

As shown in FIG. 11, the main driving circuit 200 displays waveforms of pulse wave signals detected in real time on the display window of the application program as a graphic screen. This way, a user can check the pulse wave signal detected in real time during an inaccurate pulse wave signal detection period.

The main driving circuit 200 displays a touch guide message on the application program screen so that a body part, such as the finger F or the like, may be accurately touched and the touch state may be maintained during the inaccurate pulse wave signal detection period. In this case, the pulse wave signal and touch guide message displayed in real time can be displayed by changing a warning color such as red.

Then, when the normal pulse wave signal detection period is set according to the detection result of the average magnitude value RFH of high pulses and the average magnitude value RFL of low pulses, the main driving circuit 200 displays the normal touch guide message on the application program screen so that the normal touch state may be maintained. At this time, the pulse wave signal and the touch guide message displayed in real time may be changed and displayed as a normal color such as green, blue, or the like.

In other words, the main driving circuit 200 checks and analyzes the high pulse magnitude change and the low pulse magnitude change of the pulse wave signals PPG during the biometric information measurement period to set the normal pulse wave signal detection period or the inaccurate pulse wave signal detection period in real time. In other words, the main driving circuit 200 displays a touch guide message on the application program screen so that the touch state of a body part such as the finger F can be accurately touched and maintained in place during an inaccurate pulse wave signal detection period to induce an accurate touch.

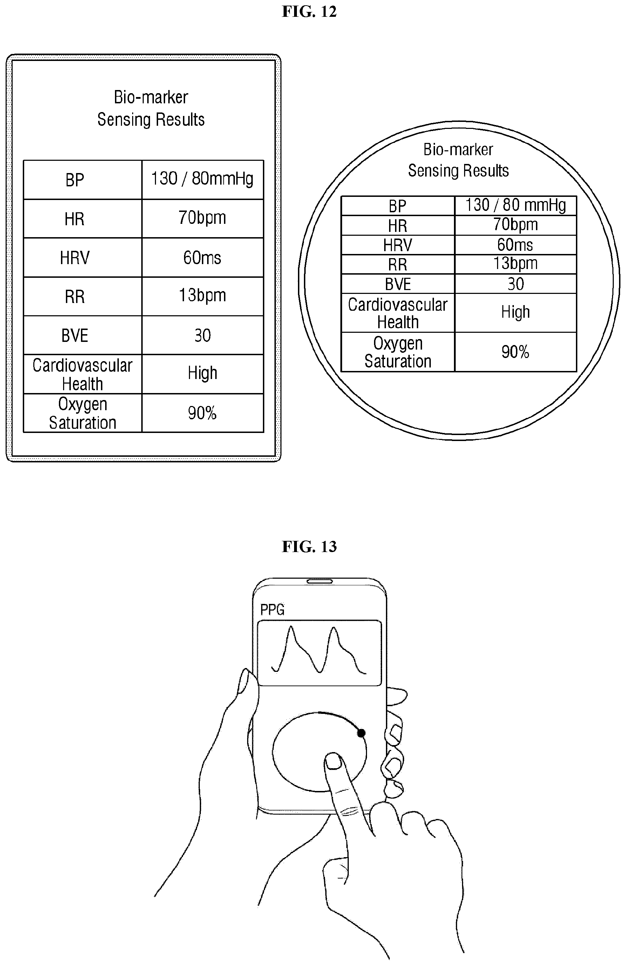

FIG. 12 is a diagram showing a biometric information measurement result through a mobile display device and a watch type display device.

Referring to FIG. 12, the display device 10 for measuring biometric information such as blood pressure, heart rate, heart rate variability, respiratory rate, blood vessel elasticity, cardiovascular disease, oxygen saturation, and the like may be applied to a mobile communication terminal such as a smart phone, a tablet display device, or the like. On the other hand, as shown in FIG. 12, the display device 10 for measuring biometric information according to an embodiment of the present disclosure may be equally applied to a wearable device such as a smart watch, a watch phone, or the like.

The main driving circuit 200 applied to a mobile communication terminal or a wearable device selects more accurate pulse wave signals PPG detected during the normal pulse wave signal detection period and measures at least one of the biometric information. The main driving circuit 200 analyzes various aspects of the detected pulse wave signals PPG during the normal pulse wave signal detection period. For example, main driving circuit 200 analyzes the high pulse cycle and the changes in the high pulse cycle, the magnitude of the high pulse and its changes, the magnitude of the low pulse and its changes, changes in the signal waveform, the period in which a high pulse reaches its peak, and the difference in the pulse magnitude of the pulse wave signals PPG respectively detected by green light and red light, among others. Then, the biometric information such as a blood pressure BP, a heart rate HR, heart rate variability HRV, a respiratory rate RR, blood vessel elasticity BVE, a cardiovascular disease (or, a cardiovascular health analysis result score), oxygen saturation, or the like is obtained according to the analysis result, and the obtained result is displayed on the application program screen.

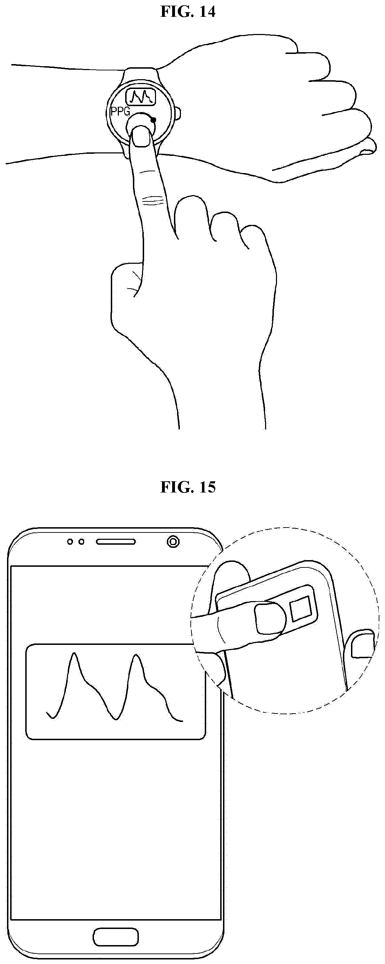

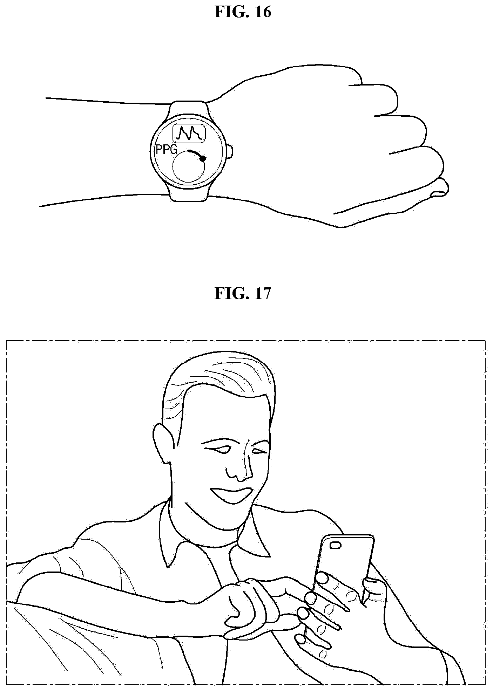

FIG. 13 is a diagram showing a biometric information measuring method using a mobile display device and an image display screen, and FIG. 14 is a diagram showing a biometric information measuring method using a watch-type display device and an image display screen. FIG. 15 is a diagram showing a biometric information measuring method using a watch-type display device with a touch sensing area formed on the rear surface of a mobile display device and an image display screen, and FIG. 16 is a diagram showing a biometric information measuring method using a touch sensing area formed on the rear surface of a watch-type display device and an image display screen. The watch-type display device may receive a light sensing signal from a user's wrist through a touch sensing area formed on the rear surface and generate pulse wave signals PPG.

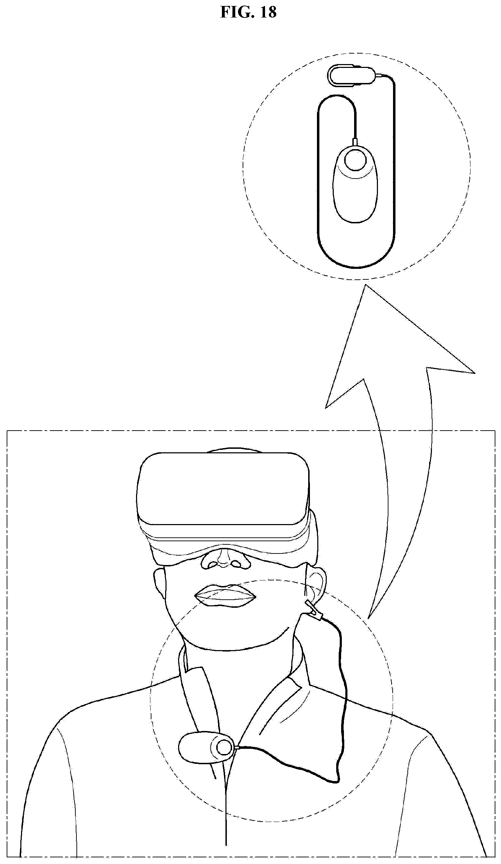

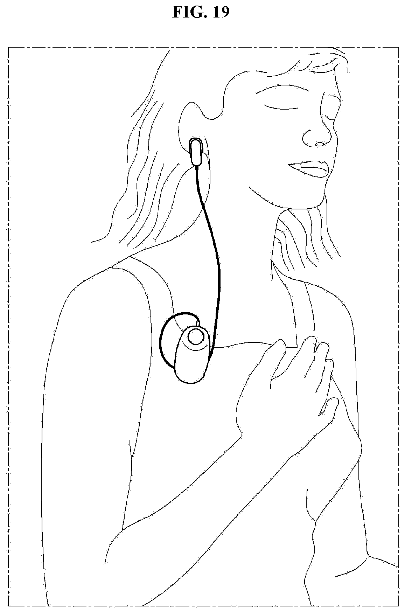

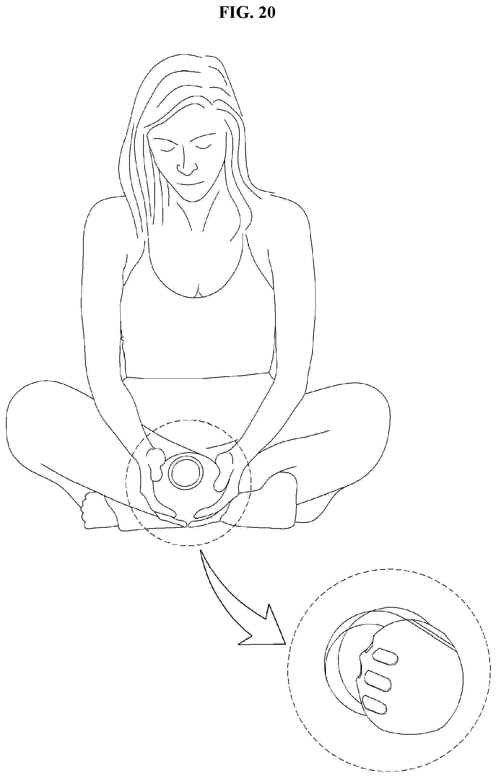

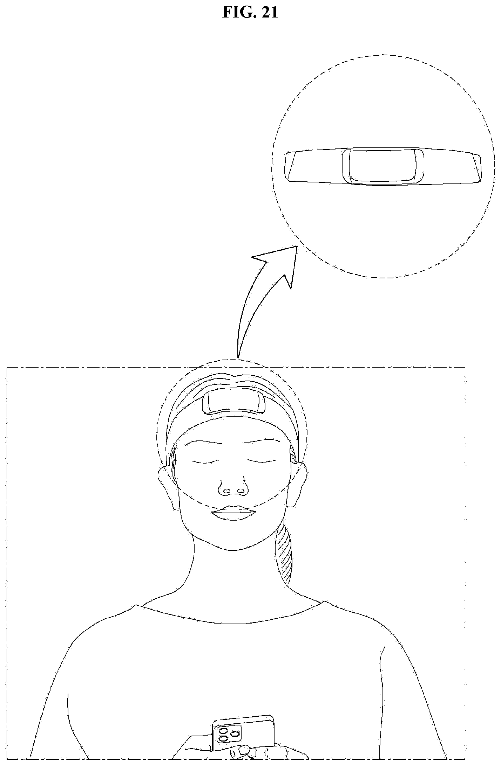

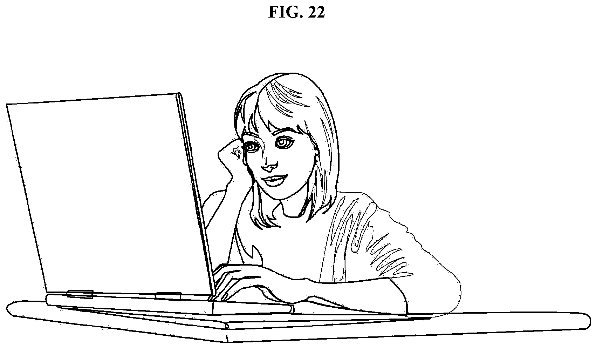

FIG. 17 is a diagram showing a biometric information measuring method using light reflected from a user's face. FIG. 18 is a diagram showing a biometric information measuring method using a pulse wave measuring module of one embodiment connected to a mobile display device in a wireless communication method. FIG. 19 is a diagram showing another biometric information measuring method using a pulse wave measuring module of one embodiment connected to a mobile display device in a wireless communication method. FIG. 20 is a diagram showing a method for measuring biometric information using a pulse wave measuring module of another embodiment connected to a mobile display device in a wireless communication method. FIG. 21 is a diagram showing a biometric information measuring method using a pulse wave measuring module of still another embodiment connected to a mobile display device in a wireless communication method. FIG. 22 is a diagram showing a biometric information measuring method using a laptop computer.

The pulse wave measuring module may be formed in a clamp type, band type, or detachable type to receive light detection signals from body parts such as a user's finger, earlobe, wrist, back of the hand, back of the foot, toe, and the like, and generate pulse wave signals PPG. Such a pulse wave measuring module may be connected to a watch-type display device, a mobile display device, or a wearable display device through a short-range wireless communication method such as Bluetooth, and transmit pulse wave signals PPG detected in real time.

Referring to FIGS. 13, 15, 17 and 22, the display device 10 for measuring biometric information such as blood pressure BP, heart rate HR, heart rate variability HRV, respiratory rate RR, blood vessel elasticity BVE, cardiovascular disease (or a cardiovascular health analysis result score), oxygen saturation, or the like may be applied to a mobile communication terminal such as a smart phone, a tablet display device, and laptop.

Further, as shown in FIGS. 14 and 16, the display device 10 for measuring biometric information may also be applied to a wearable device such as a smart watch, a watch phone, or the like. Additionally, as shown in FIGS. 18, 19, 20 and 21, the display device 10 for measuring biometric information may be applied to a wearable display device or a mobile display device connected to a separate pulse wave measurement module through a short-range wireless communication method.

The main driving circuits 200 of the display devices 10 respectively applied to the mobile display device and the watch-type display device, or the wearable device display the biometric information measurement area FSA touched by a body part such as the finger F or the like on the application program screen in the display area DA and guide the pulse wave signal detection process. Further, the pulse wave signals of the body part that has touched the biometric information measurement area FSA of the display area DA may be respectively detected and stored as digital signal data.

Referring to FIGS. 13, 15 and 17, the main driving circuit 200 of the mobile display device may store first pulse wave signals PPG detected by the biometric information measurement area FSA of the mobile as first digital signal data.

Referring to FIG. 14, the main driving circuit 200 of the watch-type display device may store second pulse wave signals PPG detected by the biometric information measurement area FSA of the watch-type display device as second digital signal data.

Referring to FIG. 15, the main driving circuit 200 may guide the pulse wave signal detection process by displaying the application program in the display area DA, and may detect the pulse wave signals PPG of a body part that has touched the biometric information measurement area FSA formed on the rear surface of the display device 10 together with a video camera or the like. The main driving circuit 200 may also store third pulse wave signals PPG detected by the biometric information measurement area FSA formed on the rear surface of the display device 10 as third digital signal data.

Further, referring to FIG. 16, the main driving circuit 200 of the watch-type display device may detect a light sensing signal and fourth pulse wave signals PPG at the user's wrist part by using the detection area formed on the rear surface of the watch-type display device. Then, the detected fourth pulse wave signals PPG may be stored as fourth digital signal data.

Further, referring to FIG. 17, the light sensing pixels LSP of the biometric information measurement area FSA may sense light reflected from the user's facial part and generate a light sensing signal, and the main driving circuit 200 may generate fifth pulse wave signals PPG corresponding to the light sensing signal sensed by using the light reflected from the facial part. Fifth pulse wave signals PPG may be stored as the fifth digital signal data.

Additionally, referring to FIGS. 18, 19, 20 and 21, the main driving circuit 200 may receive pulse wave signals PPG in a short-range wireless communication method through a separate pulse wave measuring module. The main driving circuit 200 may store each of the $n^{th}$ pulse wave signals PPG detected and received through the pulse wave measuring module as $n^{th}$ digital signal data.

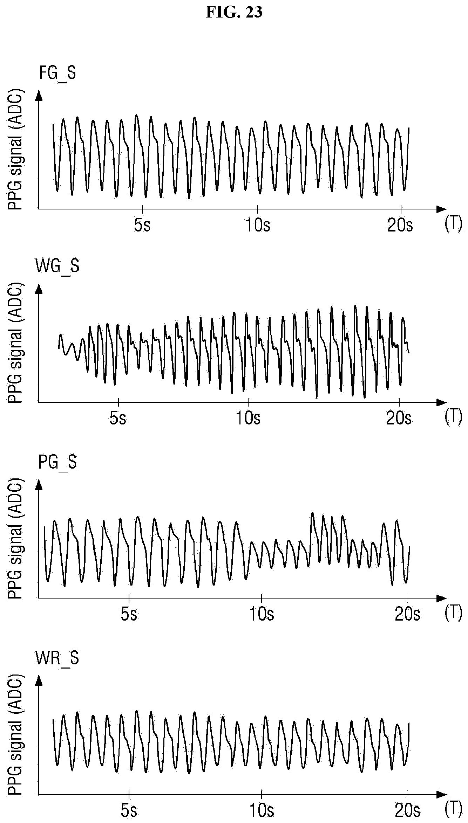

FIG. 23 is a graph showing a comparison of a mobile display device, a watch-type display device, pulse wave signals detected in the touch sensing area on the rear surface of the mobile display device, and pulse wave signals detected in the touch sensing area on the rear surface of the watch type display device.

Referring to FIG. 23, the main driving circuit 200 formed at any one display device 10, such as a mobile display device or a watch type display device, receives the pulse wave signals PPG using the main driving circuits 200 of other display devices 10 connected by a short-distance wireless communication method such as Bluetooth, Wi-Fi, mirroring, or the like.

Referring to FIGS. 13 to 22 together with FIG. 23, any one main driving circuit 200 may receive the first to $n^{th}$

digital signal data (e.g., the first to fifth digital signal data) respectively including the first to nth pulse wave signals PPG (e.g., the first to fifth pulse wave signals PPG) using other adjacent main driving circuits 200.

The main driving circuit 200 receives, compares, and analyzes the first to nth pulse wave signals received from the main driving circuit 200 of different display devices 10. Then, any one pulse wave signal PPG may be selected according to the analysis result to measure at least one piece of biometric information.

For example, the main driving circuit 200 may receive first pulse wave signals FG_S detected by the finger F that has touched the biometric information measurement area FSA of the display area DA, second pulse wave signals WG_S detected by the finger F that has touched the biometric information measurement area FSA of the watch-type display device, third pulse wave signals PG_S detected by the biometric information measurement area FSA on the rear surface of the mobile display device, and fourth pulse wave signals WR_S detected by a wrist part that has touched the detection area on the rear surface of the watch-type display device. In this case, the main driving circuit 200 may calculate the average magnitude value RFH of high pulses and the average magnitude value RFL of low pulses of each of the first to fourth pulse wave signals FG_S, WG_S, PG_S, and WR_S. The main driving circuit 200 may then compare the first to fourth pulse wave signals FG_S, WG_S, PG_S, and WR_S with each other and select one of the first to fourth pulse wave signals FG_S, WG_S, PG_S, and WR_S according to the comparison result. Then, the selected pulse wave signal may be analyzed to obtain the biometric information such as the blood pressure BP, the heart rate HR, the heart rate variability HRV, the respiratory rate RR, the blood vessel elasticity BVE, the cardiovascular disease (or the cardiovascular health analysis result score), the oxygen saturation, or the like, and the obtained result may be displayed on the application program screen.

Alternatively, the main driving circuit 200 calculates the average magnitude value RFH of high pulses and the average magnitude value RFL of low pulses of the first to fourth pulse wave signals FG_S, WG_S, PG_S, and WR_S. Then, the calculated average magnitude value RFH of high pulses is compared with the preset high threshold ThH, and the average magnitude value RFL of low pulses is compared with the preset low threshold ThL. Then, any one of the first to fourth pulse wave signals FG_S, WG_S, PG_S, and WR_S may be selected according to the comparison result. Accordingly, the main driving circuit 200 may analyze the selected pulse wave signal to obtain the biometric information such as the blood pressure BP, the heart rate HR, the heart rate variability HRV, the respiratory rate RR, the blood vessel elasticity BVE, the cardiovascular disease (or the cardiovascular health analysis result score), the oxygen saturation, or the like, and may display the obtained result on the application program screen.

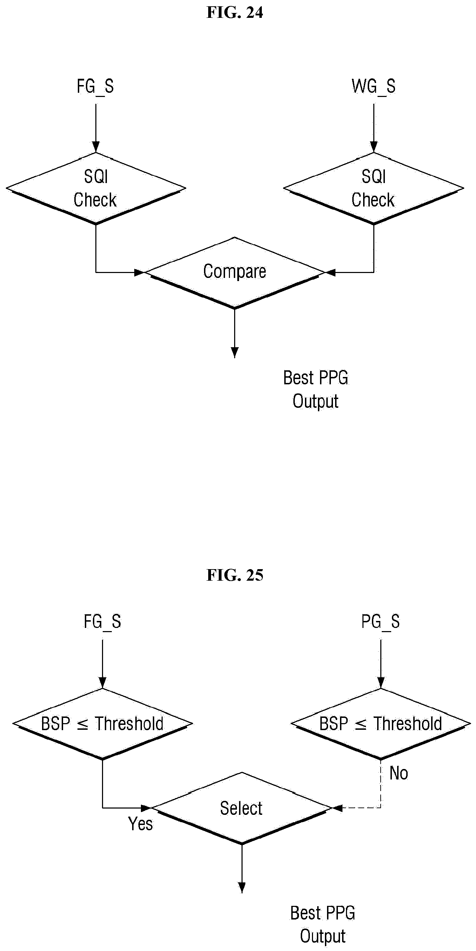

FIG. 24 is a flowchart illustrating a process of selecting a pulse wave signal for measuring biometric information by comparing pulse wave signals respectively detected in a mobile display device and a watch type display device.

Referring to FIGS. 23 and 24, the main driving circuit 200 calculates the average magnitude value RFH of high pulses and the average magnitude value RFL of low pulses of the first to fourth pulse wave signals FG_S, WG_S, PG_S, and WR_S that are respectively generated and calculated (SQI Check step).

Sequentially, the main driving circuit 200 compares the average magnitude values RFH of high pulses of the first to fourth pulse wave signals FG_S, WG_S, PG_S, and WR_S with each other to select any one pulse wave signal having the largest average magnitude value RFH of high pulses (Compare step). As an example, the first pulse wave signal FG_S that has the largest average magnitude value RFH of high pulses of the first to fourth pulse wave signals FG_S, WG_S, PG_S and WR_S may be selected. Alternatively, any one pulse wave signal having a median value among the average magnitude values RFH of high pulses that are compared with each other may be selected.

Alternatively, the main driving circuit 200 may compare the average magnitude values RFL of low pulses of the first to fourth pulse wave signals FG_S, WG_S, PG S, and WR_S with each other to select any one pulse wave signal having the smallest average magnitude value RFL of low pulses (Compare step). Alternatively, any one pulse wave signal having a median value may be selected among the average magnitude values RFL of low pulses that are compared with each other (Compare step).

Thereafter, the main driving circuit 200 may analyze the selected pulse wave signal to obtain the biometric information such as the blood pressure BP, the heart rate HR, the heart rate variability HRV, the respiratory rate RR, the blood vessel elasticity BVE, the cardiovascular disease (or the cardiovascular health analysis result score), the oxygen saturation, or the like, and may display the obtained result on the application program screen.

FIG. 25 is a flowchart illustrating a process of selecting a pulse wave signal for measuring biometric information among pulse wave signals respectively detected in the touch sensing areas on the front surface and the rear surface of a mobile display device.

Referring to FIG. 25, the main driving circuit 200 calculates the average magnitude value RFH of high pulses and the average magnitude value RFL of low pulses of the first to fourth pulse wave signals FG_S, WG_S, PG_S, and WR_S that are respectively generated and calculated.

The main driving circuit 200 compares the average magnitude value RFH of high pulses calculated for each of the first to fourth pulse wave signals FG_S, WG_S, PG_S, and WR_S with the preset high threshold ThH, and compares the average magnitude value RFL of low pulses with the preset low threshold ThL (BSP≤Threshold step).

The main driving circuit 200 may select any one pulse wave signal having the largest average magnitude value RFH of high pulses compared to the preset high threshold ThH (Select step). Alternatively, any one pulse wave signal having the largest average magnitude value RFL of low pulses compared to the preset low threshold ThL may be selected (Select step).

The main driving circuit 200 may analyze the selected pulse wave signal to obtain the biometric information such as the blood pressure BP, the heart rate HR, the heart rate variability HRV, the respiratory rate RR, the blood vessel elasticity BVE, the cardiovascular disease (or the cardiovascular health analysis result score), the oxygen saturation, or the like, and may display the obtained result on the application program screen.

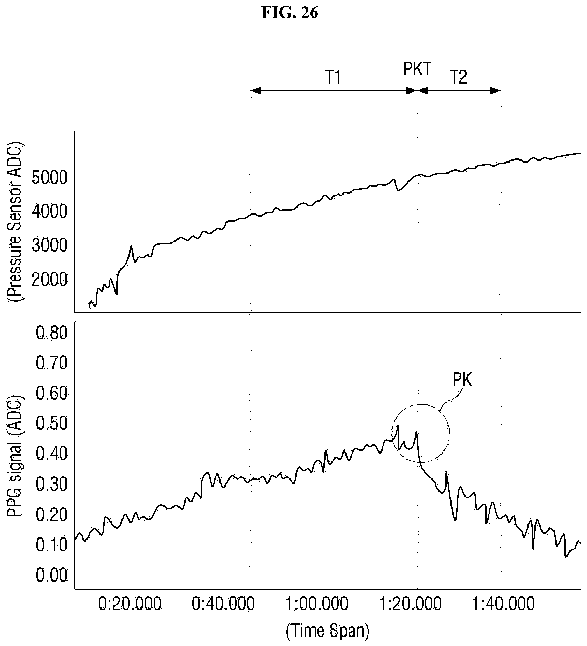

FIG. 26 is a graph illustrating a method for calculating blood pressure information among biometric information according to one embodiment.

Referring to FIG. 26, during the systole of the heart, the blood ejected from the left ventricle of the heart moves to the peripheral tissues, and thus the blood volume in the arterial side increases. Further, when the heart contracts, red blood cells carry more oxygen hemoglobin to the peripheral tissues. When the heart relaxes, the heart receives a partial influx of blood from the peripheral tissues. When light is irradiated to peripheral blood vessels, the irradiated light is absorbed by the peripheral tissues. Light absorbance depends on hematocrit and blood volume. The light absorbance may have a maximum value when the heart contracts and may have a minimum value when the heart relaxes. Therefore, light sensed by the light sensing element PD may be the least when the heart contracts and may be the most when the heart relaxes.

Further, when the user puts the finger F on the display panel 100 and lifts it off in the blood pressure measurement mode, a force (e.g., contact force) applied to the pressure sensing unit PSU may gradually increase to reach a maximum value, and then may gradually decrease. When the contact force increases, blood vessels may be narrowed, resulting in no blood flow. When the contact force decreases, the blood vessels expand, and thus blood flows again. A further decrease of the contact force results in greater blood flow. Therefore, the change in the amount of light sensed by the light sensing pixel LSP may be proportional to the change in blood flow. Accordingly, the main driving circuit 200 generates the pulse wave signals PPG according to the pressure applied by a user based on a pressure data value (e.g., ADC of the pressure sensing unit) that is calculated by the pressure sensing unit PSU and digitally converted and the optical signal (e.g., PPG signal ratio) according to the amount of light sensed by the light sensing element PD. The pulse wave signals PPG may have a waveform vibrating according to the cardiac cycle.

The main driving circuit 200 may estimate blood pressures of the blood vessels of the finger F based on time differences between time points PKT corresponding to peaks PK of the calculated pulse wave signals PPG and time points corresponding to peaks of the filtered pulse wave. For example, the main driving circuit 200 may calculate pulse wave signals during preset periods T1 and T2 before and after the time points PKT corresponding to the peaks PK of the calculated pulse wave signal, and may detect blood pressure according to differences between the pulse wave signals. Among the estimated blood pressure values, a maximum blood pressure value may be determined as a systolic blood pressure value, and a minimum blood pressure value may be determined as a diastolic blood pressure value. Further, additional blood pressure values such as an average blood pressure value or the like may be calculated using the estimated blood pressure values.

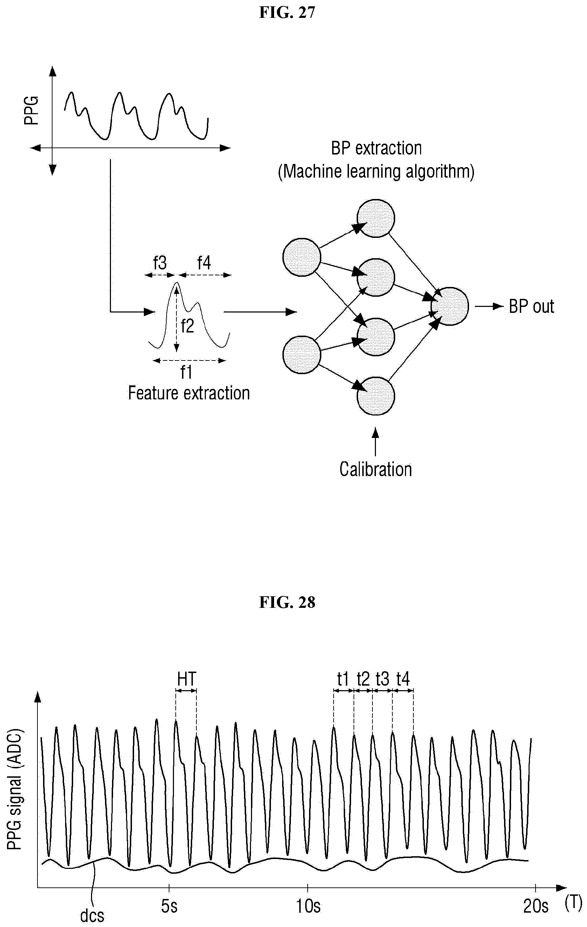

FIG. 27 is a diagram illustrating a blood pressure information calculation method using a machine learning algorithm according to another embodiment of the present disclosure.

Referring to FIG. 27, the main driving circuit 200 may learn signal features (for example, signal feature points) of the pulse wave signals PPG using a machine learning algorithm, and may detect the information on the blood pressure BP according to a signal feature change rate of the pulse wave signals PPG.

For example, the main driving circuit 200 sets an initial value or a reference value for each of a pulse width (f1, for example, systolic and diastolic cycles), an amplitude (f2, for example, systolic blood pressure), a pulse width of the systolic cycle (f3, for example, systolic cycle), a diastolic cycle (f4, for example, pulse width of the diastolic cycle) of each of the pulse wave signals PPG. Thereafter, the main driving circuit 200 detects changes in the characteristics of the pulse wave signals PPG inputted in real time, comparing them with the initial value or the reference value of each of the characteristics. For example, the main driving circuit 200 detects the magnitudes of changes in the systolic and diastolic cycles, changes in the systolic blood pressure, changes in the systolic cycle, and changes in the diastolic cycle, and stores these as learning data. The main driving circuit 200 may output the information on the blood pressure BP according to the feature change rate or the magnitude of changes of the pulse wave signals PPG compared to the initial value or the reference value of each of the characteristics.

The method for measuring the blood pressure described above is only an example. For example, various other methods are disclosed in Korean Patent Application Publication No. 10-2018-0076050, Korean Patent Application Publication No. 10-2017-0049280, and Korean Patent Application Publication No. 10-2019-0040527, the disclosures of which are incorporated by reference herein in their entireties.

FIG. 28 is a graph illustrating a method for calculating information on blood vessel elasticity among biometric information according to one embodiment.

Referring to FIG. 28, the main driving circuit 200 samples pulse wave signals during a preset sampling period before and after the time points PKT corresponding to the peaks PK of the pulse wave signal, and detects a high pulse generation cycle HT of the sampled pulse wave signals PPG. Further, the number of high pulses generated for a preset reference period (for example, 60 seconds) may be counted for the sampled pulse wave signals PPG to detect biometric information on the heart rate cycle and the heart rate HR.

Further, the main driving circuit 200 detects the heart rate cycle, which corresponds to the high pulse generation cycle HT, and the heart rate cycle changes 11, t2, t3 and t4 of high pulses for each preset reference period for the peaks PK of the pulse wave signal to detect the heart rate variability HRV according to the heart rate cycle change rate.

Additionally, the main driving circuit 200 sequentially detects the generation cycle and the magnitude value of low pulses of the sampled pulse wave signals PPG. Then, the change cycle of a magnitude value dcs of low pulses may be detected in units of preset reference periods (for example, 60 seconds) to detect the respiratory change state and the respiratory rate RR of a user. At this time, the cycle in which the magnitude value dcs of low pulses increases and the cycle in which the magnitude value dcs of low pulses decreases may be analyzed to detect the respiratory change state and the respiratory rate RR of the user using the increasing cycle and the decreasing cycle of the magnitude value dcs of low pulses.

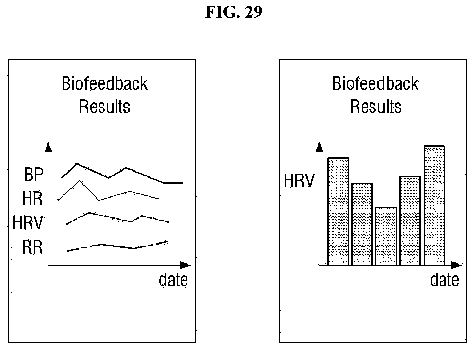

FIG. 29 is a diagram illustrating a heart rate variability change state measured in real time by feeding it back to a mobile and watch-type display device as an application program screen.

Referring to FIG. 29, the main driving circuit 200 displays at least one of the biometric information detected in real time, for example, at least one of the biometric information such as the blood pressure BP, the heart rate HR, the heart rate variability HRV, the respiratory rate RR on the application program screen.

Based on the user's option selection, the main driving circuit 200 may display at least one of the biometric information detected in real time as a linear graph image, as text on a table, or in various forms such as a bar graph.

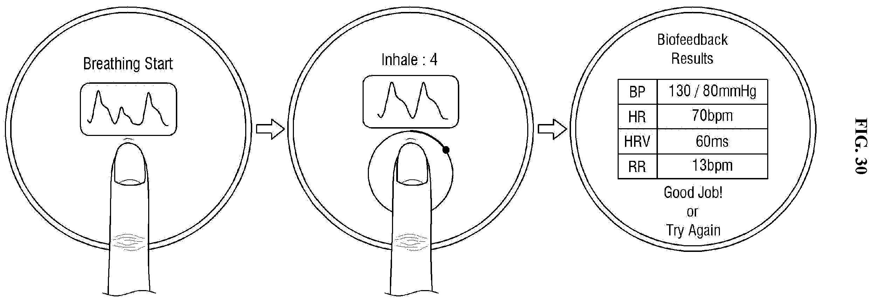

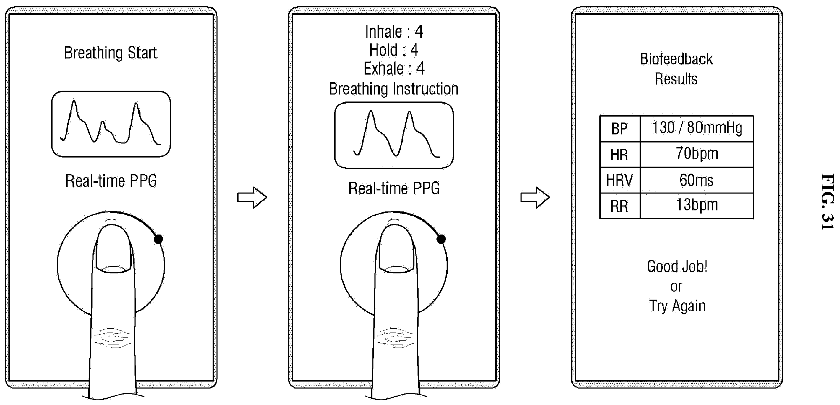

FIG. 30 is a diagram illustrating the respiratory rate and breathing timing information measured in real time by feeding them back to a watch-type display device as an application program screen. In addition, FIG. 31 is a diagram illustrating respiratory rate and breathing timing information measured in real time by feeding them back to a mobile display device as an application screen.

The main driving circuit 200 may output meditation music and display meditation screens so that the user can have a meditation time or a time to mentally and physically stabilize through a preset user behavior guide program. In other words, the main driving circuit 200 can produce meditation music and show calming visuals, allowing users to engage in meditation or take time to mentally and physically stabilize using a predefined user behavior guide program.

In addition, during the meditation period or mental and physical stabilization period, in other words, the period during which the user behavior guide program is executed, the main driving circuit 200 feeds back and displays heart rate variability, respiratory rate, and breathing timing information on the application program screen in real time.

Referring to FIGS. 30 and 31, the main driving circuit 200 supplies data voltage to the display area DA of the display panel 100 according to the user's option selection or control while the user behavior guide program is being executed and supplies control signals to the display scan driver 110 and the light sensing scan driver 120 to cause a notification screen to display the user's heart rate HR and heart rate variability HRV as feedback on the display window of the application program.

The main driving circuit 200 may display a meditation screen on at least a portion of the application screen displayed on the display area DA, and display a notification window or display window on the remaining portion of the application screen. In this case, while the meditation screen of the user behavior guide program is being executed, the main driving circuit 200 detects and counts the high pulse generation cycle HT of the pulse wave signals PPG to detect the biometric information with respect to the heart rate HR and the heart rate variability HRV. In addition, the heart rate HR and the heart rate variability HRV detected in real time are displayed in text on the display window of the biometric information notification screen. In this case, the heart rate HR and the heart rate variability HRV may also be output as audio such as sound. Accordingly, using the real-time heat rate HR and heat rate variability HRV data displayed on the application program screen, the user can adjust their breathing patterns and rhythm to achieve a more balanced mental and physical state.

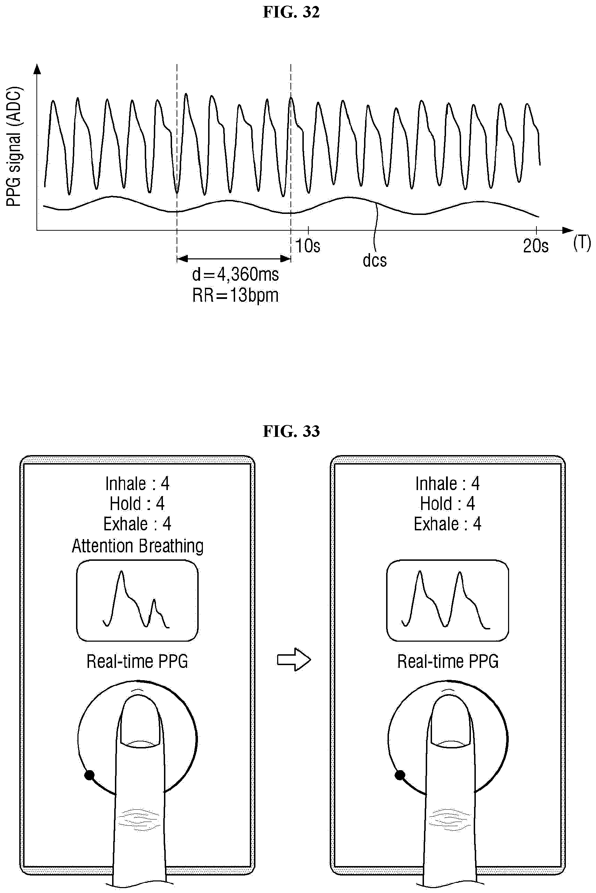

FIG. 32 is a graph illustrating a method of calculating respiratory rate and breathing timing information among biometric information according to an embodiment.

The main driving circuit 200 may display a notification screen for displaying the user's respiratory rate and breathing timing information as feedback to the user's behavior guide program in a display window while the meditation screen and sound are displayed.

While the user behavior guide program is being executed, the main driving circuit 200 sequentially detects the generation cycle of low pulses and the magnitude value of the low pulses of the pulse wave signals PPG. The main driving circuit 200 then analyzes the cycle in which the magnitude value dcs of low pulses increases and the cycle in which the magnitude value dcs of low pulses decreases to detect the breathing timing information (e.g., inhalation and exhalation status information) and respiratory rate RR. In addition, the respiratory change state and respiratory rate RR detected in real time are displayed as text on the display window of the biometric information notification screen. In this case, the respiratory change state and the respiratory rate RR may be output as sound. Accordingly, the user can adjust their respiratory rate and their breathing timing by referring to the respiration rate RR and the respiratory change state displayed in the application program screen and audio in real time.

FIG. 33 is a diagram illustrating breathing timing information fed back to a user behavior guide program screen so that breathing timing can be induced during a meditation period or mental and physical stabilization period.

Referring to FIG. 33, the main driving circuit 200 may display a breathing timing guidance screen on the display window for displaying breathing timing guidance information including the respiratory rate and the number of inhalation and exhalation periods to the user while the user behavior guide program is being executed.

The main driving circuit 200 compares the user's real-time inhalation and exhalation periods during the execution of the user behavior guide program, with a present range of inhalation and exhalation periods for meditation. The main driving circuit 200 also compares the respiratory rate RR information, in preset periods, to a standard respiratory rate range. In addition, when the user's inhalation and exhalation periods detected in real time and the respiratory rate RR in a preset period are included within the preset range of inhalation and exhalation periods for meditation and the range below or above the standard respiratory rate range, the breathing timing guide information including preset ranges of the inhalation and exhalation periods for meditation and the standard respiratory rate range is displayed in real time on the display window of the biometric information notification screen. In this case, the breathing timing including the range of inhalation period and exhalation period for meditation and the standard respiratory rate may be output as sound. Accordingly, the user can adjust the respiratory rate and breathing timing by referring to the range of the inhalation period and the exhalation period for meditation and the standard respiratory rate displayed on the application program screen and audio in real time.

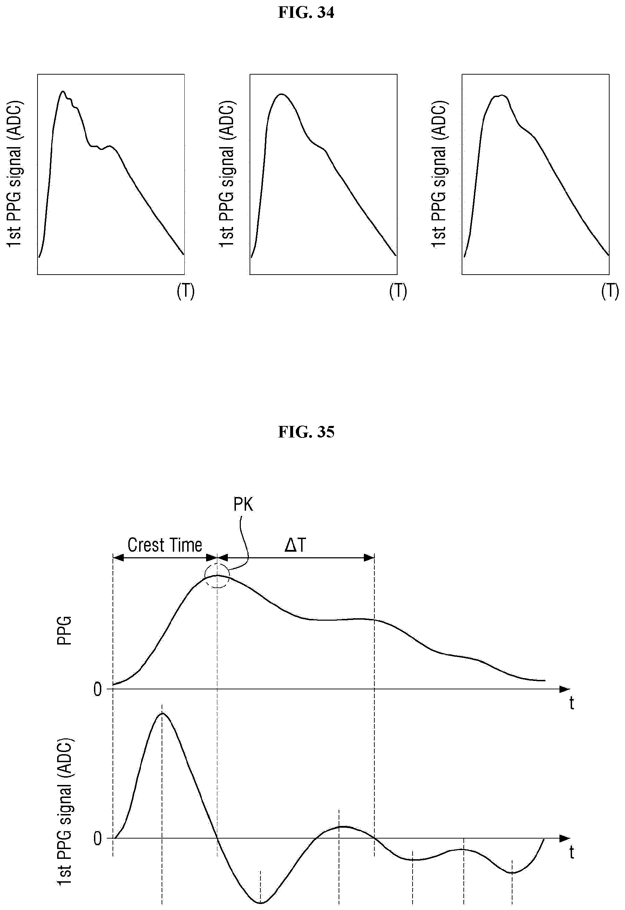

FIG. 34 is a graph illustrating a method for calculating information on blood vessel elasticity among biometric information according to one embodiment.

Referring to FIG. 34, the main driving circuit 200 may set and obtain the blood vessel elasticity BVE by expanding and analyzing the high pulse variation of the sampled pulse wave signals PPG.

When the blood flow increases due to heartbeat, the pulse wave signal is changed to a high pulse form, and when the blood flow decreases, the pulse wave signal is changed again to a low pulse form. If the blood flow changes rapidly due to the shape of the blood vessel during the period in which the blood flow increases or decreases, the change in the blood flow may be quickly relaxed or slowed depending on the elasticity of the blood vessel. Accordingly, the main driving circuit 200 sets and obtains the blood vessel elasticity BVE using a value corresponding to the magnitude of changes in high pulses by expanding and analyzing the high pulse change form of the pulse wave signals PPG.

FIG. 35 is a graph illustrating a method for calculating information on cardiovascular disease among biometric information according to one embodiment.

Referring to FIG. 35, the main driving circuit 200 may set and obtain a cardiovascular disease evaluation score (or a cardiovascular health analysis result score) by differentiating and expanding and analyzing the high pulse change form of the sampled pulse wave signals PPG. For example, the main driving circuit 200 detects a period (Crest Time) in which the pulse wave signals PPG reach the peak PK in a high pulse form, and time variation $\Delta T$ in which the pulse wave signals PPG fall compared to the period (Crest Time) in which the pulse wave signals PPG reach the peak PK. As the period (Crest Time) in which the pulse wave signals PPG reach the peak PK in a high pulse form increases, the risk of heart disease increases. Accordingly, the main driving circuit 200 may set and obtain the cardiovascular disease evaluation score (or the cardiovascular health analysis result score) in inverse proportion to the period (Crest Time) in which the pulse wave signals PPG reach the peak PK in a high pulse form.

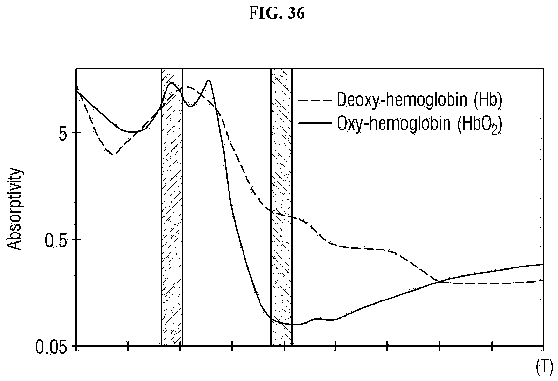

FIG. 36 is a graph illustrating a method for calculating information on oxygen saturation among biometric information according to one embodiment.

Referring to FIG. 36, when the heart contracts, red blood cells carry more oxygen hemoglobin to peripheral tissues. On the other hand, when the heart relaxes, the heart receives a partial influx of blood from the peripheral tissues. Using this, the main driving circuit 200 detects a deoxy-hemoglobin (Hb) value using the magnitude change of the pulse wave signals PPG detected by the green light, and detects a $HbO_2$(Oxy-hemoglobin) value using the magnitude change of the pulse wave signals PPG detected by the red light.

The main driving circuit 200 may detect the oxygen saturation ($SpO_2$) using the following Eq. (2).

$$SpO_2 = HpO_2 \,/\, SpO_2 + Hb \ldots\ldots\ldots\ldots\ldots(2)$$

The main driving circuit 200 may display the biometric information such as the blood pressure BP, the heart rate HR, the heart rate variability HRV, the respiratory rate RR, the blood vessel elasticity BVE, the cardiovascular disease (or the cardiovascular health analysis result score), the oxygen saturation ($SpO_2$), or the like on the application program screen.

In concluding the detailed description, those skilled in the art will appreciate that many variations and modifications can be made to the embodiments without substantially departing from the principles of the present disclosure. Therefore, the disclosed embodiments of the disclosure are used in a generic and descriptive sense and not for purposes of limitation.

**Claims**

1. A display device comprising:

   a display panel (100) having a display area (DA);
   display pixels (SP) arranged in the display area ;
   light sensing pixels (LSP) arranged in the display area;
   a display scan driver (110) configured to drive the display pixels to emit light;
   a light sensing scan driver (120) configured to drive the light sensing pixels to sense light; and
   a main driving circuit (200) configured to measure a user's biometric information using light sensing signals received from the light sensing pixels,
   wherein the main driving circuit (200) is configured to display a preset user behavior guidance program screen during a measurement period of biometric information to guide a user's biometric signal detection process, and display user behavior guidance information and the biometric information measurement result using the biometric signal in real time.

2. The display device of claim 1,

   wherein the main driving circuit (200) is configured to detect pulse wave signals among the user's biometric signals by using the light sensing signals during the measurement period of biometric information,
   analyze a high pulse cycle and a high pulse cycle change of the pulse wave signals, a high pulse magnitude and a magnitude change of the pulse wave signals, a low pulse magnitude and a magnitude change of the pulse wave signals, a high pulse waveform change, a period in which the high pulse reaches its peak, and the difference in magnitude of the pulse wave signals respectively detected by green light and red light, and measure at least one of biometric information among blood pressure, heart rate, heart rate variability, respiratory rate, blood vessel elasticity, a cardiovascular disease analysis result, and oxygen saturation according to each analysis result and displays the biometric information on the user behavior guidance program screen.

3. The display device of claim 1 or 2,

   wherein the main driving circuit (200) is configured to display meditation-related user behavior guidance information on the user behavior guidance program screen, and
   a notification screen for displaying the user's heart rate and heart rate variability is displayed when the meditation-related user behavior guidance information is displayed.

4. The display device of claim 3,

   wherein the main driving circuit (200) is configured to detect and count a generation cycle of the high pulses of the pulse wave signals to detect the user's heart rate and heart rate variability in real time, and
   display the user's heart rate and heart rate variability in real time.

5. The display device of claim 2,
   wherein the main driving circuit (200) is configured to display a meditation screen on the user behavior guidance program screen, output sound, and provide feedback for the user's respiratory rate and breathing timing information during a period in which the meditation screen is displayed.

6. The display device of claim 5,

   wherein the main driving circuit (200) is configured to sequentially detect a generation cycle and the magnitude of the low pulses of the pulse wave signals,
   analyze a cycle in which the magnitude of the low pulses increases and a cycle in which the magnitude of the low pulses decreases to detect the user's breathing timing information and the respiratory rate, and
   display the user's breathing timing information and the respiratory rate in real time.

**7.** The display device of claim 2,
wherein the main driving circuit (200) is configured to display a breathing timing guidance screen on a display window for showing breathing timing guidance information including a respiratory rate and inhalation and exhalation periods to a user during the period in which the user behavior guidance program is being executed.

**8.** The display device of claim 7,

wherein the main driving circuit (200) is configured to compare the user's inhalation and exhalation period detected in real time during the execution period of the user behavior guidance program and respiratory rate to a preset range for the inhalation period and exhalation period for meditation and a range for a standard respiratory rate, respectively, and
when the user's inhalation and exhalation period and the respiratory rate are included within the preset range for the inhalation period and exhalation period for meditation and the range below the standard respiratory rate, breathing timing guidance information including preset ranges for the inhalation period and exhalation period for meditation and the standard respiratory rate is displayed in real time on the display window.

**9.** The display device of claim 2,

wherein the main driving circuit (200) is configured to display a touch sensing area of the user's body part while the user behavior guidance program screen is displayed on the display area,
display waveforms of the pulse wave signals detected in real time on a display window of the user behavior guidance program screen to guide the user's pulse wave signal detection process, and
display a detection period of the pulse wave signals and a period during which the pulse wave signals need to be detected to adequately measure the biometric information.

**10.** The display device of claim 9,

wherein the main driving circuit (200) is configured to generate the pulse wave signals corresponding to magnitudes of the light sensing signals and changes in the magnitudes of the light sensing signals,
analyze the high pulse magnitude change and the low pulse magnitude change of the pulse wave signals to calculate an average magnitude value of the high pulses and an average magnitude value of the low pulses in real time, and
set a normal pulse wave signal detection period or an inaccurate pulse wave signal detection period in real time according to a comparison result of the average magnitude value of the high pulses to a preset high threshold and the comparison result of the average magnitude value of the low pulses to a preset low threshold, and/or
wherein the main driving circuit is configured to set the inaccurate pulse wave signal detection period when the average magnitude value of the high pulses is smaller than the preset high threshold during a plurality of preset frame periods, or
when the average magnitude value of the low pulses is smaller than the preset low threshold during the plurality of preset frame periods.

**11.** The display device of claim 10,

wherein the main driving circuit (200) is configured to generate a capacitance profile or an image based on values of the light sensing signals detected by the light sensing pixels, detect a touch area of the user's body part based on the capacitance profile or the image, and set an incorrect pulse wave signal detection period when the detected touch area is smaller than a reference area, and/or
wherein the main driving circuit (200) is configured to display a first touch guide message on the user behavior guidance program screen so that the user's body part can be accurately touched and a touch state can be maintained during the inaccurate pulse wave signal detection period, and
display the waveforms of the pulse wave signals in real time on the display window and the touch guide message indicating a warning.

**12.** The display device of claim 11,

wherein the main driving circuit (200) is configured to display a second touch guide message on the user behavior guidance program screen so that a normal touch state can be maintained when the normal pulse wave signal detection period is set, and

display the waveforms of the pulse wave signals in real time on the display window and the touch guide message indicating a normal condition.

**13.** The display device of claim 2,

wherein the main driving circuit (200) is configured to generate first pulse wave signals corresponding to magnitudes of the light sensing signals and changes in the magnitudes of the light sensing signals,
receive at least one second pulse wave signal or an $n^{th}$ pulse wave signal from a main driving circuit formed in at least one other adjacent display device,
compare and analyzes the first and second pulse wave signals or at least two pulse wave signals among the first to $n^{th}$ pulse wave signals, and measures the biometric information by selecting and using any one pulse wave signal according to the analysis result.

**14.** The display device of claim 13,
wherein the main driving circuit (200) is configured to calculate and compares an average magnitude value of high pulses and an average magnitude value of low pulses of the first and second pulse wave signals or the first to $n^{th}$ pulse wave signals, selects any one of the pulse wave signals according to the comparison result, and measures the biometric information by analyzing the selected pulse wave signal.

**15.** The display device of claim 14,

wherein the main driving circuit (200) is configured to compare average magnitude values of high pulses of the first and second pulse wave signals or the first to $n^{th}$ pulse wave signals with each other, and selects any one pulse wave signal having the largest average magnitude value of the high pulses to measure the biometric information,
measure the biometric information by selecting any one pulse wave signal having a median value among the average magnitude values of the high pulses compared with each other,
compare average magnitude values of low pulses of the first and second pulse wave signals or the first to $n^{th}$ pulse wave signals with each other, and selects any one pulse wave signal having the smallest average magnitude value of the low pulses to measure the biometric information, or
measure the biometric information by selecting any one pulse wave signal having a median value among the average magnitude values of the low pulses compared with each other, and/or
wherein the main driving circuit is configured to calculate an average magnitude value of high pulses and an average magnitude value of low pulses of the first and second pulse wave signals or the first to $n^{th}$ pulse wave signals,
compares the calculated average magnitude value of the high pulses with a preset high threshold, compares the average magnitude value of the low pulses with a preset low threshold, selects any one of the pulse wave signals according to the comparison result, and measures the biometric information by analyzing the selected pulse wave signal.

**FIG. 1**

FIG. 2

## FIG. 3

## FIG. 4

**FIG. 5**

## FIG. 6

PG1 : SP1, SP2, SP3, LSP
PG2 : SP1, SP2, SP3, ISP

**FIG. 7**

ELU: LEL
DDU: ST1~ST7, DT, CST1

## FIG. 8

Good

Real-time PPG

FSA

F

## FIG. 9

**FIG. 10**

ODC image based area calculation

(a)     (b)     (c)

Cap profile based area calculation

(a)     (b)     (c)

**FIG. 11**

Bad(Warning)

Place finger well

FSA

F

**FIG. 12**

Bio-marker
Sensing Results

| BP | 130 / 80mmHg |
|---|---|
| HR | 70bpm |
| HRV | 60ms |
| RR | 13bpm |
| BVE | 30 |
| Cardiovascular Health | High |
| Oxygen Saturation | 90% |

Bio-marker
Sensing Results

| BP | 130 / 80 mmHg |
|---|---|
| HR | 70bpm |
| HRV | 60ms |
| RR | 13bpm |
| BVE | 30 |
| Cardiovascular Health | High |
| Oxygen Saturation | 90% |

**FIG. 13**

PPG

**FIG. 14**

**FIG. 15**

FIG. 16

FIG. 17

**FIG. 18**

**FIG. 19**

**FIG. 20**

**FIG. 21**

FIG. 22

**FIG. 23**

FG_S

WG_S

PG_S

WR_S

FIG. 24

FG_S                                                    WG_S

SQI
Check                                                   SQI
                                                        Check

Compare

Best PPG
Output

FIG. 25

FG_S                                                    PG_S

BSP ≤ Threshold                                         BSP ≤ Threshold

                                                                          No

Select

Yes

Best PPG
Output

FIG. 26

**FIG. 27**

**FIG. 28**

**FIG. 29**

**FIG. 30**

**FIG. 31**

**FIG. 32**

d = 4,360ms
RR = 13bpm

**FIG. 33**

**FIG. 34**

**FIG. 35**

FIG. 36

Europäisches
Patentamt
European
Patent Office
Office européen
des brevets

# EUROPEAN SEARCH REPORT

**Application Number**

EP 24 17 0970

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | EP 4 298 993 A1 (SAMSUNG DISPLAY CO LTD [KR]) 3 January 2024 (2024-01-03) | 1 | INV. A61B5/021 A61B5/024 A61B5/00 G06F3/00 |
| A,P | * abstract * * paragraph [0005] - paragraph [0007] * * paragraph [0037] - paragraph [0083] * * paragraph [0201] - paragraph [0239] * * figures 1,2,5,18-27 * | 2 | |
| A | US 2021/267550 A1 (MUKKAMALA RAMAKRISHNA [US] ET AL) 2 September 2021 (2021-09-02) * paragraph [0070] - paragraph [0073] * * paragraph [0098] - paragraph [0113] * * figures 4B,7 * | 1 | |
| A | US 11 617 539 B2 (SAMSUNG ELECTRONICS CO LTD [KR]) 4 April 2023 (2023-04-04) * column 2, line 16 - line 25 * * column 10, line 20 - column 14, line 51 * * column 27, line 4 - column 28, line 32 * * figures 2,12,13 * | 2-7 | |
| A | US 11 179 047 B2 (UNIV MICHIGAN STATE [US]; UNIV MARYLAND [US]) 23 November 2021 (2021-11-23) * abstract * * column 3, line 20 - line 52 * * column 5, line 24 - column 7, line 19 * * column 8, line 4 - line 50 * * figures 1,2C * | 2 | **TECHNICAL FIELDS SEARCHED (IPC)** A61B G06F |

The present search report has been drawn up for all claims

| Place of search | Date of completion of the search | Examiner |
|---|---|---|
| The Hague | 9 September 2024 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 0970

This annex lists the patent family members relating to the patent documents cited in the above-mentioned European search report.
The members are as contained in the European Patent Office EDP file on
The European Patent Office is in no way liable for these particulars which are merely given for the purpose of information.

09-09-2024

| Patent document cited in search report | | Publication date | Patent family member(s) | | Publication date |
|---|---|---|---|---|---|
| EP 4298993 | A1 | 03-01-2024 | CN | 117297565 A | 29-12-2023 |
| | | | CN | 220327453 U | 12-01-2024 |
| | | | EP | 4298993 A1 | 03-01-2024 |
| | | | KR | 20240003020 A | 08-01-2024 |
| | | | US | 2024000389 A1 | 04-01-2024 |
| US 2021267550 | A1 | 02-09-2021 | EP | 3813653 A1 | 05-05-2021 |
| | | | US | 2021267550 A1 | 02-09-2021 |
| | | | WO | 2020006518 A1 | 02-01-2020 |
| US 11617539 | B2 | 04-04-2023 | CN | 105380597 A | 09-03-2016 |
| | | | CN | 111317449 A | 23-06-2020 |
| | | | EP | 2992817 A1 | 09-03-2016 |
| | | | EP | 4018919 A1 | 29-06-2022 |
| | | | KR | 20220093306 A | 05-07-2022 |
| | | | US | 2016058376 A1 | 03-03-2016 |
| | | | US | 2020077952 A1 | 12-03-2020 |
| | | | US | 2022047215 A1 | 17-02-2022 |
| | | | WO | 2016036114 A1 | 10-03-2016 |
| US 11179047 | B2 | 23-11-2021 | US | 2019008399 A1 | 10-01-2019 |
| | | | US | 2020008693 A1 | 09-01-2020 |
| | | | US | 2021298618 A1 | 30-09-2021 |

EPO FORM P0459

For more details about this annex : see Official Journal of the European Patent Office, No. 12/82

**REFERENCES CITED IN THE DESCRIPTION**

**Patent documents cited in the description**

- KR 1020180076050
- KR 1020170049280
- KR 1020190040527